(19) **Europäisches Patentamt**

**European Patent Office**

**Office européen des brevets**

(11) **EP 1 357 127 A1**

(12) **EUROPÄISCHE PATENTANMELDUNG**

(43) Veröffentlichungstag:
**29.10.2003 Patentblatt 2003/44**

(21) Anmeldenummer: **02008033.9**

(22) Anmeldetag: **10.04.2002**

(51) Int Cl.[7]: **C07K 14/18**, A61K 39/29,
G01N 33/576, G01N 33/68,
C12N 15/51, A61K 48/00,
A61P 31/14

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(71) Anmelder: **Immusystems GmbH
80538 München (DE)**

(72) Erfinder:
• **Diepolder, Helmut, Dr.
80689 München (DE)**
• **Jung, Maria-Christina, Dr.
80538 München (DE)**

(74) Vertreter: **Grünecker, Kinkeldey,
Stockmair & Schwanhäusser Anwaltssozietät
Maximilianstrasse 58
80538 München (DE)**

(54) **CD4+ T-Lymphozyten spezifische Hepatitis C Virus-Epitope**

(57) Die Erfindung betrifft Hepatitis C Virus-Epitope, die gegenüber CD4[+] T-Lymphozyten spezifisch sind, sowie Impfstoffe, die diese Epitope enthalten.

EP 1 357 127 A1

SEQUENZPROTOKOLL

<110> Immusystems GmbH

<120> CD4+Lymphozyten spezifische HCV EPITOPE

<130> EP23423-099

<160> 140

<170> PatentIn version 3.1

<210> 1
<211> 15
<212> PRT
<213> Hepatitis C virus

<400> 1

Asp Val Lys Phe Pro Gly Gly Gly Gln Ile Val Gly Gly Val Tyr
1               5                   10                  15


<210> 2
<211> 15
<212> PRT
<213> Hepatitis C virus

<400> 2

Gly Pro Arg Leu Gly Val Arg Ala Thr Arg Lys Thr Ser Glu Arg
1               5                   10                  15


<210> 3
<211> 15
<212> PRT
<213> Hepatitis C virus

<400> 3

Arg Arg Gln Pro Ile Pro Lys Ala Arg Arg Pro Glu Gly Arg Ala
1               5                   10                  15


<210> 4
<211> 15
<212> PRT
<213> Hepatitis C virus

<400> 4

Pro Glu Gly Arg Ala Trp Ala Gln Pro Gly Tyr Pro Trp Pro Leu
1               5                   10                  15


<210> 5
<211> 15
<212> PRT
<213> Hepatitis C virus

```
<400>  5

Tyr Pro Trp Pro Leu Tyr Gly Asn Glu Gly Leu Gly Trp Ala Gly
1               5                   10                  15


<210>  6
<211>  15
<212>  PRT
<213>  Hepatitis C virus

<400>  6

Arg Gly Ser Arg Pro Ser Trp Gly Pro Thr Asp Pro Arg Arg Arg
1               5                   10                  15


<210>  7
<211>  15
<212>  PRT
<213>  Hepatitis C virus

<400>  7

Gly Ala Pro Leu Gly Gly Ala Ala Arg Ala Leu Ala His Gly Val
1               5                   10                  15


<210>  8
<211>  15
<212>  PRT
<213>  Hepatitis C virus

<400>  8

Leu Ala His Gly Val Arg Val Leu Glu Asp Gly Val Asn Tyr Ala
1               5                   10                  15


<210>  9
<211>  15
<212>  PRT
<213>  Hepatitis C virus

<400>  9

Gly Val Asn Tyr Ala Thr Gly Asn Leu Pro Gly Cys Ser Phe Ser
1               5                   10                  15


<210>  10
<211>  15
<212>  PRT
<213>  Hepatitis C virus

<400>  10

Ala Tyr Glu Val Arg Asn Val Ser Gly Val Tyr His Val Thr Asn
1               5                   10                  15
```

```
<210>  11
<211>  15
<212>  PRT
<213>  Hepatitis C virus

<400>  11

Tyr His Val Thr Asn Asp Cys Ser Asn Ser Ser Ile Val Tyr Glu
1               5                   10                  15


<210>  12
<211>  15
<212>  PRT
<213>  Hepatitis C virus

<400>  12

Ser Ile Val Tyr Glu Ala Ala Asp Met Ile Met His Thr Pro Gly
1               5                   10                  15


<210>  13
<211>  15
<212>  PRT
<213>  Hepatitis C virus

<400>  13

Met His Thr Pro Gly Cys Val Pro Cys Val Arg Glu Asn Asn Ser
1               5                   10                  15


<210>  14
<211>  15
<212>  PRT
<213>  Hepatitis C virus

<400>  14

Val Gln Asp Cys Asn Cys Ser Ile Tyr Pro Gly His Val Ser Gly
1               5                   10                  15


<210>  15
<211>  15
<212>  PRT
<213>  Hepatitis C virus

<400>  15

Pro Gln Ala Val Val Asp Met Val Ala Gly Ala His Trp Gly Val
1               5                   10                  15


<210>  16
<211>  15
<212>  PRT
<213>  Hepatitis C virus

<400>  16
```

```
Tyr Tyr Ser Met Val Gly Asn Trp Ala Lys Val Leu Ile Val Met
1               5                   10                  15
```

```
<210>   17
<211>   15
<212>   PRT
<213>   Hepatitis C virus

<400>   17
```

```
Val Asp Gly Gly Gly Gly Thr Thr His Val Thr Gly Gly Ala Ala
1               5                   10                  15
```

```
<210>   18
<211>   15
<212>   PRT
<213>   Hepatitis C virus

<400>   18
```

```
Gly Phe Thr Ser Leu Phe Ser Pro Gly Ala Ser Gln Lys Ile Gln
1               5                   10                  15
```

```
<210>   19
<211>   15
<212>   PRT
<213>   Hepatitis C virus

<400>   19
```

```
Gly Ser Trp His Ile Asn Arg Thr Ala Leu Asn Cys Asn Asp Ser
1               5                   10                  15
```

```
<210>   20
<211>   15
<212>   PRT
<213>   Hepatitis C virus

<400>   20
```

```
Asn Cys Asn Asp Ser Leu Gln Thr Gly Phe Leu Ala Ala Leu Phe
1               5                   10                  15
```

```
<210>   21
<211>   15
<212>   PRT
<213>   Hepatitis C virus

<400>   21
```

```
Leu Ala Ala Leu Phe Tyr Thr His Arg Phe Asn Ser Ser Gly Cys
1               5                   10                  15
```

```
<210>   22
<211>   15
<212>   PRT
```

```
<213>  Hepatitis C virus

<400>  22

Ser Cys Arg Pro Ile Asp Lys Phe Ala Gln Gly Trp Gly Pro Ile
1               5                  10                  15


<210>  23
<211>  15
<212>  PRT
<213>  Hepatitis C virus

<400>  23

Gly Asn Trp Phe Gly Cys Thr Trp Met Asn Ser Thr Gly Phe Thr
1               5                  10                  15


<210>  24
<211>  15
<212>  PRT
<213>  Hepatitis C virus

<400>  24

Ser Thr Gly Phe Thr Lys Thr Cys Gly Gly Pro Pro Cys Asn Ile
1               5                  10                  15


<210>  25
<211>  15
<212>  PRT
<213>  Hepatitis C virus

<400>  25

Pro Pro Cys Asn Ile Gly Gly Val Gly Asn Asn Thr Leu Thr Cys
1               5                  10                  15


<210>  26
<211>  15
<212>  PRT
<213>  Hepatitis C virus

<400>  26

Asn Thr Leu Thr Cys Pro Thr Asp Cys Phe Arg Lys His Pro Glu
1               5                  10                  15


<210>  27
<211>  15
<212>  PRT
<213>  Hepatitis C virus

<400>  27

Arg Lys His Pro Glu Ala Thr Tyr Thr Lys Cys Gly Ser Gly Pro
1               5                  10                  15
```

```
<210>  28
<211>  15
<212>  PRT
<213>  Hepatitis C virus

<400>  28

Cys Gly Ser Gly Pro Trp Leu Thr Pro Arg Cys Leu Val Asp Tyr
1                   5                   10                  15


<210>  29
<211>  15
<212>  PRT
<213>  Hepatitis C virus

<400>  29

Thr Gly Leu Ile His Leu His Gln Asn Ile Val Asp Val Gln Tyr
1                   5                   10                  15


<210>  30
<211>  15
<212>  PRT
<213>  Hepatitis C virus

<400>  30

Gln Tyr Phe Ile Thr Arg Ala Glu Ala His Leu Gln Val Trp Val
1                   5                   10                  15


<210>  31
<211>  15
<212>  PRT
<213>  Hepatitis C virus

<400>  31

Arg Gly Gly Arg Asp Ala Ile Ile Leu Leu Thr Cys Ala Val His
1                   5                   10                  15


<210>  32
<211>  15
<212>  PRT
<213>  Hepatitis C virus

<400>  32

Thr Arg Val Pro Tyr Phe Val Arg Ala Gln Gly Leu Ile Arg Ala
1                   5                   10                  15


<210>  33
<211>  15
<212>  PRT
<213>  Hepatitis C virus

<400>  33
```

```
Gly Leu Ile Arg Ala Cys Met Leu Val Arg Lys Val Ala Gly Gly
1                   5                   10                  15
```

<210> 34
<211> 15
<212> PRT
<213> Hepatitis C virus

<400> 34

```
Ala Phe Met Lys Leu Ala Ala Leu Thr Gly Thr Tyr Val Tyr Asp
1                   5                   10                  15
```

<210> 35
<211> 15
<212> PRT
<213> Hepatitis C virus

<400> 35

```
Ser Asp Met Glu Thr Lys Ile Ile Thr Trp Gly Ala Asp Thr Ala
1                   5                   10                  15
```

<210> 36
<211> 15
<212> PRT
<213> Hepatitis C virus

<400> 36

```
Ile Leu Gly Leu Pro Val Ser Ala Arg Arg Gly Arg Glu Ile Leu
1                   5                   10                  15
```

<210> 37
<211> 15
<212> PRT
<213> Hepatitis C virus

<400> 37

```
Ser Leu Glu Gly Gln Gly Trp Arg Leu Leu Ala Pro Ile Thr Ala
1                   5                   10                  15
```

<210> 38
<211> 15
<212> PRT
<213> Hepatitis C virus

<400> 38

```
Leu Thr Gly Arg Asp Lys Asn Gln Val Glu Gly Glu Val Gln Val
1                   5                   10                  15
```

<210> 39
<211> 15

23

```
<212>   PRT
<213>   Hepatitis C virus

<400>   39

Gly Glu Val Gln Val Val Ser Thr Ala Thr Gln Ser Phe Leu Ala
1                   5                   10                  15


<210>   40
<211>   15
<212>   PRT
<213>   Hepatitis C virus

<400>   40

Ile Thr Gln Met Tyr Thr Asn Val Asp Gln Asp Leu Val Gly Trp
1                   5                   10                  15


<210>   41
<211>   15
<212>   PRT
<213>   Hepatitis C virus

<400>   41

Asp Leu Val Gly Trp Gln Ala Pro Pro Gly Ala Arg Ser Leu Thr
1                   5                   10                  15


<210>   42
<211>   15
<212>   PRT
<213>   Hepatitis C virus

<400>   42

Ala Arg Ser Leu Thr Pro Cys Thr Cys Gly Ser Ser Asp Leu Tyr
1                   5                   10                  15


<210>   43
<211>   15
<212>   PRT
<213>   Hepatitis C virus

<400>   43

Ser Ser Asp Leu Tyr Leu Val Thr Arg His Ala Asp Val Ile Pro
1                   5                   10                  15


<210>   44
<211>   15
<212>   PRT
<213>   Hepatitis C virus

<400>   44

Ala Asp Val Ile Pro Val Arg Arg Arg Gly Asp Ser Arg Gly Ser
1                   5                   10                  15
```

```
<210>   45
<211>   15
<212>   PRT
<213>   Hepatitis C virus

<400>   45

Pro Val Ser Tyr Leu Lys Gly Ser Ser Gly Gly Pro Leu Leu Cys
1                   5                   10                  15


<210>   46
<211>   15
<212>   PRT
<213>   Hepatitis C virus

<400>   46

His Leu His Ala Pro Thr Gly Ser Gly Lys Ser Thr Lys Val Pro
1                   5                   10                  15


<210>   47
<211>   15
<212>   PRT
<213>   Hepatitis C virus

<400>   47

Ser Thr Lys Val Pro Ala Ala Tyr Ala Ala Gln Gly Tyr Lys Val
1                   5                   10                  15


<210>   48
<211>   15
<212>   PRT
<213>   Hepatitis C virus

<400>   48

Gln Gly Tyr Lys Val Leu Val Leu Asn Pro Ser Val Ala Ala Thr
1                   5                   10                  15


<210>   49
<211>   15
<212>   PRT
<213>   Hepatitis C virus

<400>   49

Asn Ile Arg Thr Gly Val Arg Thr Ile Thr Thr Gly Ala Pro Ile
1                   5                   10                  15


<210>   50
<211>   15
<212>   PRT
<213>   Hepatitis C virus
```

```
<400>  50

Thr Gly Ala Pro Ile Thr Tyr Ser Thr Tyr Gly Lys Phe Leu Ala
1               5                   10                  15


<210>  51
<211>  15
<212>  PRT
<213>  Hepatitis C virus

<400>  51

Val Pro His Pro Asn Ile Glu Glu Val Ala Leu Ser Asn Thr Gly
1               5                   10                  15


<210>  52
<211>  15
<212>  PRT
<213>  Hepatitis C virus

<400>  52

Leu Ser Asn Thr Gly Glu Ile Pro Phe Tyr Gly Lys Ala Ile Pro
1               5                   10                  15


<210>  53
<211>  20
<212>  PRT
<213>  Hepatitis C virus

<400>  53

Ala Ile Pro Leu Glu Val Ile Lys Gly Gly Arg His Leu Ile Phe Cys
1               5                   10                  15


His Ser Lys Arg
            20


<210>  54
<211>  15
<212>  PRT
<213>  Hepatitis C virus

<400>  54

Gly Lys Ala Ile Pro Ile Glu Val Ile Lys Gly Gly Arg His Leu
1               5                   10                  15


<210>  55
<211>  15
<212>  PRT
<213>  Hepatitis C virus

<400>  55

Gly Gly Arg His Leu Ile Phe Cys His Ser Lys Lys Lys Cys Asp
```

1           5                   10                  15

```
<210>  56
<211>  15
<212>  PRT
<213>  Hepatitis C virus

<400>  56

Lys Lys Lys Cys Asp Glu Leu Ala Ala Lys Leu Ser Ala Leu Gly
1               5               10              15
```

```
<210>  57
<211>  20
<212>  PRT
<213>  Hepatitis C virus

<400>  57

Lys Leu Val Ala Met Gly Ile Asn Ala Val Ala Tyr Tyr Arg Gly Leu
1               5               10              15

Asp Val Ser Val
            20
```

```
<210>  58
<211>  15
<212>  PRT
<213>  Hepatitis C virus

<400>  58

Leu Ser Ala Leu Gly Leu Asn Ala Val Ala Tyr Tyr Arg Gly Leu
1               5               10              15
```

```
<210>  59
<211>  15
<212>  PRT
<213>  Hepatitis C virus

<400>  59

Tyr Tyr Arg Gly Leu Asp Val Ser Val Ile Pro Thr Ser Gly Asp
1               5               10              15
```

```
<210>  60
<211>  20
<212>  PRT
<213>  Hepatitis C virus

<400>  60

Ser Val Ile Pro Thr Ser Gly Asp Val Val Val Ala Thr Asp Ala
1               5               10              15
```

```
Leu Met Thr Gly
            20
```

```
<210>  61
<211>  15
<212>  PRT
<213>  Hepatitis C virus

<400>  61

Asp Pro Thr Phe Thr Ile Glu Thr Thr Thr Val Pro Gln Asp Ala
1               5                   10                  15
```

```
<210>  62
<211>  15
<212>  PRT
<213>  Hepatitis C virus

<400>  62

Gly Ile Tyr Arg Phe Val Thr Pro Gly Glu Arg Pro Ser Gly Met
1               5                   10                  15
```

```
<210>  63
<211>  15
<212>  PRT
<213>  Hepatitis C virus

<400>  63

Arg Pro Ser Gly Met Phe Asp Ser Ser Val Leu Cys Glu Cys Tyr
1               5                   10                  15
```

```
<210>  64
<211>  18
<212>  PRT
<213>  Hepatitis C virus

<400>  64

Thr Thr Val Arg Leu Arg Ala Tyr Met Asn Thr Pro Gly Leu Pro Val
1               5                   10                  15


Cys Gln
```

```
<210>  65
<211>  15
<212>  PRT
<213>  Hepatitis C virus

<400>  65

Val Arg Leu Arg Ala Tyr Leu Asn Thr Pro Gly Leu Pro Val Cys
1               5                   10                  15
```

```
<210>   66
<211>   20
<212>   PRT
<213>   Hepatitis C virus

<400>   66

Gln Thr Lys Gln Ser Gly Glu Asn Leu Pro Tyr Leu Val Ala Tyr Gln
1               5                   10                  15

Ala Thr Val Cys
            20


<210>   67
<211>   20
<212>   PRT
<213>   Hepatitis C virus

<400>   67

Tyr Leu Val Ala Tyr Gln Ala Thr Val Cys Ala Arg Ala Gln Ala Pro
1               5                   10                  15

Pro Pro Ser Trp
            20


<210>   68
<211>   20
<212>   PRT
<213>   Hepatitis C virus

<400>   68

Ala Arg Ala Gln Ala Pro Pro Pro Ser Trp Asp Gln Met Trp Lys Cys
1               5                   10                  15

Leu Ile Arg Leu
            20


<210>   69
<211>   15
<212>   PRT
<213>   Hepatitis C virus

<400>   69

Ala Gln Ala Pro Pro Pro Ser Trp Asp Gln Met Trp Lys Cys Leu
1               5                   10                  15


<210>   70
<211>   20
<212>   PRT
<213>   Hepatitis C virus
```

<400> 70

```
Asp Gln Met Trp Lys Cys Leu Ile Arg Leu Lys Pro Thr Leu His Gly
1               5                   10                  15

Pro Thr Pro Leu
            20
```

<210> 71
<211> 15
<212> PRT
<213> Hepatitis C virus

<400> 71

```
Met Trp Lys Cys Leu Ile Arg Leu Lys Pro Thr Leu His Gly Pro
1               5                   10                  15
```

<210> 72
<211> 15
<212> PRT
<213> Hepatitis C virus

<400> 72

```
Thr Ser Thr Trp Val Leu Val Gly Gly Val Leu Ala Ala Leu Ala
1               5                   10                  15
```

<210> 73
<211> 15
<212> PRT
<213> Hepatitis C virus

<400> 73

```
Ile Leu Ser Gly Arg Pro Ala Val Ile Pro Asp Arg Glu Val Leu
1               5                   10                  15
```

<210> 74
<211> 15
<212> PRT
<213> Hepatitis C virus

<400> 74

```
Tyr Ile Glu Gln Gly Met Gln Leu Ala Glu Gln Phe Lys Gln Lys
1               5                   10                  15
```

<210> 75
<211> 15
<212> PRT
<213> Hepatitis C virus

<400> 75

```
Ala Pro Val Val Glu Ser Lys Trp Arg Ala Leu Glu Thr Phe Trp
```

```
1                  5                      10                     15
```

```
<210>  76
<211>  20
<212>  PRT
<213>  Hepatitis C virus

<400>  76

Gln Lys Leu Glu Thr Phe Trp Ala Lys His Met Trp Asn Phe Ile Ser
1                  5                      10                     15


Gly Ile Gln Tyr
            20


<210>  77
<211>  20
<212>  PRT
<213>  Hepatitis C virus

<400>  77

Met Trp Asn Phe Ile Ser Gly Ile Gln Tyr Leu Ala Gly Leu Ser Thr
1                  5                      10                     15


Leu Pro Gly Asn
            20


<210>  78
<211>  15
<212>  PRT
<213>  Hepatitis C virus

<400>  78

Asn Phe Ile Ser Gly Ile Gln Tyr Leu Ala Gly Leu Ser Thr Leu
1                  5                      10                     15


<210>  79
<211>  20
<212>  PRT
<213>  Hepatitis C virus

<400>  79

Leu Ala Gly Leu Ser Thr Leu Pro Gly Asn Pro Ala Ile Ala Ser Leu
1                  5                      10                     15


Met Ala Phe Thr
            20


<210>  80
<211>  15
<212>  PRT
```

<213> Hepatitis C virus

<400> 80

Gly Leu Ser Thr Leu Pro Gly Asn Pro Ala Ile Ala Ser Leu Met
1               5               10                  15


<210> 81
<211> 20
<212> PRT
<213> Hepatitis C virus

<400> 81

Gln Thr Leu Leu Phe Asn Ile Leu Gly Gly Trp Val Ala Ala Gln Leu
1               5               10                  15


Ala Ala Pro Gly
            20


<210> 82
<211> 15
<212> PRT
<213> Hepatitis C virus

<400> 82

Leu Leu Phe Asn Ile Leu Gly Gly Trp Val Ala Ala Gln Leu Ala
1               5               10                  15


<210> 83
<211> 15
<212> PRT
<213> Hepatitis C virus

<400> 83

Ala Ala Gln Leu Ala Pro Pro Ser Ala Ala Ser Ala Phe Val Gly
1               5               10                  15


<210> 84
<211> 15
<212> PRT
<213> Hepatitis C virus

<400> 84

Ser Ala Phe Val Gly Ala Gly Ile Ala Gly Ala Ala Val Gly Ser
1               5               10                  15


<210> 85
<211> 15
<212> PRT
<213> Hepatitis C virus

<400> 85

```
Val Leu Val Asp Ile Leu Ala Gly Tyr Gly Ala Gly Val Ala Gly
1               5                   10                  15


<210>  86
<211>  15
<212>  PRT
<213>  Hepatitis C virus

<400>  86

Lys Val Met Ser Gly Glu Met Pro Ser Thr Glu Asp Leu Val Asn
1               5                   10                  15


<210>  87
<211>  15
<212>  PRT
<213>  Hepatitis C virus

<400>  87

Glu Asp Leu Val Asn Leu Leu Pro Ala Ile Leu Ser Pro Gly Ala
1               5                   10                  15


<210>  88
<211>  20
<212>  PRT
<213>  Hepatitis C virus

<400>  88

Thr His Tyr Val Pro Glu Ser Asp Ala Ala Ala Arg Val Thr Gln Ile
1               5                   10                  15

Leu Ser Ser Leu
            20


<210>  89
<211>  20
<212>  PRT
<213>  Hepatitis C virus

<400>  89

Thr Ile Thr Gln Leu Leu Lys Arg Leu His Gln Trp Ile Asn Glu Asp
1               5                   10                  15

Cys Ser Thr Pro
            20


<210>  90
<211>  15
<212>  PRT
<213>  Hepatitis C virus
```

33

<400> 90

Leu Lys Arg Leu His Gln Trp Ile Asn Glu Asp Cys Ser Thr Pro
1               5                   10                  15

<210> 91
<211> 20
<212> PRT
<213> Hepatitis C virus

<400> 91

Cys Ser Gly Ser Trp Leu Arg Asp Val Trp Asp Trp Ile Cys Thr Val
1               5                   10                  15

Leu Thr Asp Phe
          20

<210> 92
<211> 15
<212> PRT
<213> Hepatitis C virus

<400> 92

Leu Arg Asp Val Trp Asp Trp Ile Cys Thr Val Leu Thr Asp Phe
1               5                   10                  15

<210> 93
<211> 15
<212> PRT
<213> Hepatitis C virus

<400> 93

Val Leu Thr Asp Phe Lys Thr Trp Leu Gln Ser Lys Leu Leu Pro
1               5                   10                  15

<210> 94
<211> 15
<212> PRT
<213> Hepatitis C virus

<400> 94

Ser Lys Leu Leu Pro Arg Leu Pro Gly Val Pro Phe Phe Ser Cys
1               5                   10                  15

<210> 95
<211> 15
<212> PRT
<213> Hepatitis C virus

<400> 95

Gly Val Trp Arg Gly Asp Gly Ile Met Gln Thr Thr Cys Pro Cys

1                    5                        10                        15

<210> 96
<211> 20
<212> PRT
<213> Hepatitis C virus

<400> 96

Asp Gly Ile Met Gln Thr Thr Cys Pro Cys Gly Ala Gln Ile Thr Gly
1                    5                        10                        15

His Val Lys Asn
               20

<210> 97
<211> 15
<212> PRT
<213> Hepatitis C virus

<400> 97

Thr Thr Cys Pro Cys Gly Ala Gln Ile Thr Gly His Val Lys Asn
1                    5                        10                        15

<210> 98
<211> 20
<212> PRT
<213> Hepatitis C virus

<400> 98

Gly Ala Gln Ile Thr Gly His Val Lys Asn Gly Ser Met Arg Ile Val
1                    5                        10                        15

Gly Pro Lys Thr
               20

<210> 99
<211> 15
<212> PRT
<213> Hepatitis C virus

<400> 99

Asn Tyr Ser Arg Ala Leu Trp Arg Val Ala Ala Glu Glu Tyr Val
1                    5                        10                        15

<210> 100
<211> 20
<212> PRT
<213> Hepatitis C virus

<400> 100

```
Leu Trp Arg Val Ala Ala Glu Glu Tyr Val Glu Val Thr Arg Val Gly
1               5                   10                  15

Asp Phe His Tyr
            20


<210>  101
<211>  15
<212>  PRT
<213>  Hepatitis C virus

<400>  101

Ala Glu Glu Tyr Val Glu Val Thr Arg Val Gly Asp Phe His Tyr
1               5                   10                  15


<210>  102
<211>  20
<212>  PRT
<213>  Hepatitis C virus

<400>  102

Glu Val Thr Arg Val Gly Asp Phe His Tyr Val Thr Gly Met Thr Thr
1               5                   10                  15

Asp Asn Val Lys
            20


<210>  103
<211>  20
<212>  PRT
<213>  Hepatitis C virus

<400>  103

Cys Pro Cys Gln Val Pro Ala Pro Glu Phe Phe Thr Glu Val Asp Gly
1               5                   10                  15

Val Arg Leu His
            20


<210>  104
<211>  15
<212>  PRT
<213>  Hepatitis C virus

<400>  104

Pro Ala Pro Glu Phe Phe Thr Glu Val Asp Gly Val Arg Leu His
1               5                   10                  15


<210>  105
<211>  20
```

```
<212>  PRT
<213>  Hepatitis C virus

<400>  105

Phe Thr Glu Val Asp Gly Val Arg Leu His Arg Tyr Ala Pro Ala Cys
1               5                   10                  15


Lys Pro Leu Leu
            20


<210>  106
<211>  15
<212>  PRT
<213>  Hepatitis C virus

<400>  106

Gly Val Arg Leu His Arg Tyr Ala Pro Ala Cys Lys Pro Leu Leu
1               5                   10                  15


<210>  107
<211>  20
<212>  PRT
<213>  Hepatitis C virus

<400>  107

Arg Glu Glu Val Thr Phe Gln Val Gly Leu Asn Gln Tyr Leu Val Gly
1               5                   10                  15


Ser Gln Leu Pro
            20


<210>  108
<211>  15
<212>  PRT
<213>  Hepatitis C virus

<400>  108

Phe Gln Val Gly Leu Asn Gln Tyr Leu Val Gly Ser Gln Leu Pro
1               5                   10                  15


<210>  109
<211>  20
<212>  PRT
<213>  Hepatitis C virus

<400>  109

Thr Ser Met Leu Thr Asp Pro Ser His Ile Thr Ala Glu Thr Ala Lys
1               5                   10                  15


Arg Arg Leu Ala
```

20

<210> 110
<211> 15
<212> PRT
<213> Hepatitis C virus

<400> 110

Asp Pro Ser His Ile Thr Ala Glu Thr Ala Lys Arg Arg Leu Ala
1               5                   10                  15


<210> 111
<211> 15
<212> PRT
<213> Hepatitis C virus

<400> 111

Lys Arg Arg Leu Ala Arg Gly Ser Gly Val Pro Pro Ser Leu Ala
1               5                   10                  15


<210> 112
<211> 15
<212> PRT
<213> Hepatitis C virus

<400> 112

Pro Pro Ser Leu Ala Ser Ser Ser Ala Ser Gln Leu Ser Ala Pro
1               5                   10                  15


<210> 113
<211> 20
<212> PRT
<213> Hepatitis C virus

<400> 113

Ser Ser Ser Ala Ser Gln Leu Ser Ala Pro Ser Leu Lys Ala Thr Cys
1               5                   10                  15

Thr Thr His His
              20


<210> 114
<211> 15
<212> PRT
<213> Hepatitis C virus

<400> 114

Gln Leu Ser Ala Pro Ser Leu Lys Ala Thr Cys Thr Thr His His
1               5                   10                  15

```
<210>   115
<211>   20
<212>   PRT
<213>   Hepatitis C virus

<400>   115

Ser Leu Lys Ala Thr Cys Thr Thr His His Ser Tyr Asn Leu Asp Ser
1                   5                   10                  15

Pro Asp Ala Asp
            20


<210>   116
<211>   15
<212>   PRT
<213>   Hepatitis C virus

<400>   116

Val Ile Leu Asp Ser Phe Asp Pro Leu Arg Ala Glu Glu Asp Glu
1                   5                   10                  15


<210>   117
<211>   20
<212>   PRT
<213>   Hepatitis C virus

<400>   117

Phe Asp Pro Leu Arg Ala Glu Glu Asp Glu Arg Glu Val Ser Val Ala
1                   5                   10                  15

Ala Glu Ile Leu
            20


<210>   118
<211>   20
<212>   PRT
<213>   Hepatitis C virus

<400>   118

Arg Glu Val Ser Val Ala Ala Glu Ile Leu Arg Lys Ser Arg Lys Phe
1                   5                   10                  15

Pro Pro Ala Met
            20


<210>   119
<211>   20
<212>   PRT
<213>   Hepatitis C virus

<400>   119
```

```
Pro Ile Trp Ala Arg Pro Asp Tyr Asn Pro Pro Leu Leu Glu Ser Trp
1                   5                   10                  15

Lys Asp Pro Asp
            20
```

<210> 120
<211> 20
<212> PRT
<213> Hepatitis C virus

<400> 120

```
Pro Leu Leu Glu Ser Trp Lys Asp Pro Asp Tyr Val Pro Pro Val Val
1                   5                   10                  15

His Gly Cys Pro
            20
```

<210> 121
<211> 20
<212> PRT
<213> Hepatitis C virus

<400> 121

```
Tyr Val Pro Pro Val Val His Gly Cys Pro Leu Pro Pro Thr Lys Ala
1                   5                   10                  15

Pro Pro Ile Pro
            20
```

<210> 122
<211> 15
<212> PRT
<213> Hepatitis C virus

<400> 122

```
Gln Ala Ser Asp Asp Gly Asp Lys Gly Ser Asp Val Glu Ser Tyr
1                   5                   10                  15
```

<210> 123
<211> 20
<212> PRT
<213> Hepatitis C virus

<400> 123

```
Asp Val Val Cys Cys Ser Met Ser Tyr Thr Trp Thr Gly Ala Leu Ile
1                   5                   10                  15

Thr Pro Cys Ala
```

20

```
<210>  124
<211>  20
<212>  PRT
<213>  Hepatitis C virus

<400>  124

Trp Thr Gly Ala Leu Ile Thr Pro Cys Ala Ala Glu Glu Ser Lys Leu
1               5                   10                  15


Pro Ile Asn Ala
            20


<210>  125
<211>  15
<212>  PRT
<213>  Hepatitis C virus

<400>  125

Glu Thr Pro Ile Asp Thr Thr Ile Met Ala Lys Asn Glu Val Phe
1               5                   10                  15


<210>  126
<211>  15
<212>  PRT
<213>  Hepatitis C virus

<400>  126

Lys Asn Glu Val Phe Cys Val Gln Pro Glu Lys Gly Gly Arg Lys
1               5                   10                  15


<210>  127
<211>  15
<212>  PRT
<213>  Hepatitis C virus

<400>  127

Lys Gly Gly Arg Lys Pro Ala Arg Leu Ile Val Phe Pro Asp Leu
1               5                   10                  15


<210>  128
<211>  15
<212>  PRT
<213>  Hepatitis C virus

<400>  128

Thr Leu Pro Gln Ala Val Met Gly Ser Ser Tyr Gly Phe Gln Tyr
1               5                   10                  15
```

```
<210>   129
<211>   15
<212>   PRT
<213>   Hepatitis C virus

<400>   129

Thr Arg Cys Phe Asp Ser Thr Val Thr Glu Asn Asp Ile Arg Val
1                   5                   10                  15


<210>   130
<211>   15
<212>   PRT
<213>   Hepatitis C virus

<400>   130

Gln Cys Cys Asp Leu Ala Pro Glu Ala Arg Gln Ala Ile Arg Ser
1                   5                   10                  15


<210>   131
<211>   15
<212>   PRT
<213>   Hepatitis C virus

<400>   131

Tyr Ile Gly Gly Pro Leu Thr Asn Ser Lys Gly Gln Asn Cys Gly
1                   5                   10                  15


<210>   132
<211>   15
<212>   PRT
<213>   Hepatitis C virus

<400>   132

Gly Gln Asn Cys Gly Tyr Arg Arg Cys Arg Ala Ser Gly Val Leu
1                   5                   10                  15


<210>   133
<211>   15
<212>   PRT
<213>   Hepatitis C virus

<400>   133

Val Tyr Tyr Leu Thr Arg Asp Pro Thr Thr Pro Leu Ala Arg Ala
1                   5                   10                  15


<210>   134
<211>   15
<212>   PRT
<213>   Hepatitis C virus

<400>   134
```

```
Gln Ile Ile Gln Arg Leu His Gly Leu Ser Ala Phe Ser Leu His
1               5                   10                  15


<210>  135
<211>  15
<212>  PRT
<213>  Hepatitis C virus

<400>  135

Ala Phe Ser Leu His Ser Tyr Ser Pro Gly Glu Ile Asn Arg Val
1               5                   10                  15


<210>  136
<211>  15
<212>  PRT
<213>  Hepatitis C virus

<400>  136

Glu Ile Asn Arg Val Ala Ser Cys Leu Arg Lys Leu Gly Val Pro
1               5                   10                  15


<210>  137
<211>  15
<212>  PRT
<213>  Hepatitis C virus

<400>  137

Lys Leu Gly Val Pro Pro Leu Arg Val Trp Arg His Arg Ala Arg
1               5                   10                  15


<210>  138
<211>  15
<212>  PRT
<213>  Hepatitis C virus

<400>  138

Cys Gly Lys Tyr Leu Phe Asn Trp Ala Val Arg Thr Lys Leu Lys
1               5                   10                  15


<210>  139
<211>  15
<212>  PRT
<213>  Hepatitis C virus

<400>  139

Arg Thr Lys Leu Lys Leu Thr Pro Ile Pro Ala Ala Ser Gln Leu
1               5                   10                  15


<210>  140
<211>  11
<212>  PRT
```

```
<213>   Hepatitis C virus

<400>   140

Val Gly Val Gly Ile Tyr Leu Leu Pro Asn Arg
1               5                   10
```

**Beschreibung**

Die Erfindung betrifft Hepatitis C Virus-Epitope, die gegenüber CD4$^+$ T-Lymphozyten spezifisch sind, sowie Impfstoffe, die diese Epitope enthalten.

Das Hepatitis C Virus, im nachstehenden HCV genannt, wurde 1989 identifiziert und ist ein RNA Virus aus der Familie der Flaviviridae. Es besteht aus einem RNA-Einzelstrang von ca. 9400 Nukleotiden, die ein ca. 3000 Aminosäuren langes Vorläuferpolyprotein kodieren. Dieses Polyprotein wird in einem offenen Leserahmen translatiert und posttranslationell proteolytisch gespalten. Das Virus ist hochvariabel, und es existieren verschiedene Virusisolate, die als Genotypen bezeichnet werden und deren geographische Verteilung sehr unterschiedlich ist. Mehr als sechs Genotypen werden heute weltweit unterschieden. Diese Genotypen wiederum werden in Subtypen unterteilt. Die genetische Variabilität besteht interindividuell und intraindividuell (innerhalb eines infizierten Individuums). Die intraindividuellen Subtypen sind die sogenannten HCV Quasispezies, verwandte aber unterschiedliche Virussequenzen, die bei ungenauer Replikation entstehen.

Mit einer Prävalenz von ca. ein bis drei Prozent weltweit ist Hepatitis C eine der bedeutendsten chronischen Virusinfektionen. Man geht derzeit von mindestens 180 Mio. infizierten Individuen aus. Nach Berechnungen der Centers of Disease Control in den USA wird es aufgrund der langen Latenzzeit nach der Infektion mit dem HCV außerdem noch zu einem Anstieg der Hepatitis C assoziierten Erkrankungen bis zum Jahre 2010 kommen.

Das HCV wird überwiegend parenteral übertragen und war bis zu seiner Entdeckung die Hauptursache für die Posttransfusionshepatitis NonA-NonB. Durch routinemäßiges Testen aller Blutprodukte mit HCV-Antikörpertests der 2. und 3. Generation hat die Zahl der Posttransfusionshepatitiden drastisch abgenommen. Die sogenannte sporadische Hepatitis C sowie i.v.-Drogenmissbrauch gelten heute als Hauptübertragungswege neuer HCV Infektionen. Es sind derzeit keine Maßnahmen bekannt, um Neuinfektionen auf diesen Wegen wirksam zu verhindern.

Das HCV verursacht eine chronische Leberentzündung (Hepatitis), die im langjährigem Verlauf zu weiteren Komplikationen, wie einer Leberzirrhose, führen kann. Im Rahmen einer jahrelang bestehenden Leberzirrhose kommt es bei ca. 5% aller Infizierten zur Entwicklung eines hepatozellulären Karzinoms. In der westlichen Welt nimmt die Hepatitis C deshalb den ersten Rang als Indikation für die Lebertransplantation ein. Die Kosten für das Gesundheitswesen durch diese Transplantationen sind erheblich.

Bei chronischer Hepatitis C sind zwar gegen fast alle Virusproteine Antikörper nachweisbar, es gibt im Gegensatz zur Hepatitis B jedoch keine Anti-HCV-Antikörperkonstellation, die eine Immunität gegenüber HCV oder eine Ausheilung anzeigt. Auch die Präsenz von Antikörpern gegen das HCV während einer chronischen HCV Infektion mildert den Verlauf nicht. Im Gegenteil scheint eine erfolgreiche Therapie mit einem Absinken der Antikörpertiter verbunden zu sein. Daher ist es nicht möglich, eine Infektion mit Hepatitis C durch eine konventionelle, prophylaktische Impfung mit Hüllprotein, wie sie bei der Hepatitis B erfolgreich durchgeführt wird, zu verhindern. Eine prophylaktische Impfung ist daher derzeit nicht verfügbar.

Die einzige derzeit zugelassene Therapie der chronischen Hepatitis C ist eine Behandlung mit Interferon alpha allein oder in Kombination mit Ribavirin für 6 bis 12 Monate. Diese Therapieform ist sehr kostenintensiv, mit erheblichen Nebenwirkungen belastet und führt nur in ca. 40% der Fälle zu einer dauerhaften Viruselimination.

Hinzu kommt, dass bisherige systematische Untersuchungen im wesentlichen auf Daten basieren, die an Patienten mit einer chronischen Hepatitis C Infektion gewonnen wurden, und eine spontane Elimination des Virus zu dem Zeitpunkt, an dem bereits eine chronische HCV Infektion vorliegt, äußerst selten vorkommt. Epitope, die an Patienten mit einer chronischen HCV Infektion gefunden wurden sind daher nicht mit der Ausheilung der Erkrankung assoziiert.

Daher liegt der vorliegenden Erfindung die Aufgabe zugrunde, gegenüber CD4$^+$ T-Lymphozyten spezifische HCV-Epitope zu charakterisieren und die durch den CD4+ T-Lymphozyten identifizierten HCV-Epitope für einen Impfstoff zur Prophylaxe und/oder Therapie einer HCV-Infektion zur Verfügung zu stellen.

Die Erfindung basiert auf der besonderen Auswahl des untersuchten Patientenkollektives sowie der Selektion der getesteten HCV Peptide und der statistischen Auswertung der Ergebnisse.

Die Lösung der Aufgabe sind CD4$^+$ T-Lymphozyten spezifische HCV-Epitope
mit einem Impact factor (IF) > Mittelwert (MW)

wobei

$$IF = \frac{n_1 * 1 + n_2 * 1{,}5}{m} * 100 \qquad \text{(Formel 1)}$$

wobei
$n_1$ der Summe der Reaktionen mit $3 < SI < 6$,
$n_2$ der Summe der Reaktionen mit $SI \geq 6$ und

m der Anzahl der Tests gegen das jeweilige Peptid entspricht, wobei m ≥15 ist, und MW der Mittelwert aller Impact factoren ist.

Vorzugsweise haben die erfindungsgemäßen Epitope zumindest eine zweimalige signifikante Reaktion (d.h. SI > 3) bei einer Testanzahl m ≥ 15.

Im Sinne der vorliegenden Erfindung bedeutet "gegenüber CD4⁺ T-Lymphozyten spezifische HCV-Epitope" eine definierte Region eines Hepatitis C Proteins, die auf Grund ihrer Struktur in die komplementäre Bindungsstelle eines CD4+ T-Lymphozyten-Rezeptors "passt" und dadurch hochspezifisch eine Reaktion auslöst.

Im Sinne der vorliegenden Erfindung bedeutet "SI" den Stimulationsindex wie bekanntermaßen bestimmt.

Des weiteren wird der Mittelwert "MW" aus den Impact Factoren der jeweils getesteten Peptide, wobei jeder Impact Factor nach Formel 1 bestimmt wurde, berechnet. Die getesteten Peptide sind ausgewählt aus allen möglichen Peptiden des HCV Virus. Vorzugsweise werden die getesteten Peptide ausgewählt aus 15- bis 20meren Peptiden des HCV Virus, , die jeweils um 5 Aminosäuren überlappen und das gesamte HCV Virus abdecken. Insbesondere bevorzugt werden die getesteten Peptide ausgewählt aus dem NS3-NS4 Bereich des Virus, wobei 20mere mit 10 Aminosäuren langen überlappenden Bereichen getestet wurden.

**Kollektiv:**

Es wurde ein besonderes Patientenkollektiv gewählt und dieses auf für die Ausheilung der Erkrankung relevanten HCV spezifischen CD4+ T-Zell Epitope untersucht. Diese Patienten wurden in einer frühen (d.h. kurze Zeit nach Infektion, "akute HCV"), immunologisch aktiven Phase der Erkrankung erfaßt. Hier läßt sich der natürliche, immunologisch relevante Verlauf der Erkrankung vor allem hinsichtlich der entscheidenden zellulären Immunantwort des Körpers gegen das Virus gut untersuchen. Dies hat herausragende Bedeutung, da es nur in dieser Phase ungefähr der Hälfte der Patienten gelingt das Virus temporär zu kontrollieren oder dauerhaft zu eliminieren.

Hingegen stellt eine spontane Elimination des Virus zu einem späteren Zeitpunkt (chronische HCV) eine Rarität dar. Auch findet sich in der späten, chronischen Phase nur eine geringe oder nicht detektierbare, gegen das Virus gerichtete CD4+ T-Zellantwort. Von herausragender Wichtigkeit sind daher vor allem Epitope, die mit temporärer oder dauerhafter Viruskontrolle einher gehen. Diese Epitope bzw. die gemessene Reaktion auf diese Peptide sind also mittelbar oder unmittelbar mit der Ausheilung der HCV Infektion assoziiert und stellen somit ideale Kandidaten für zukünftige "Peptidimpfungen" dar. Dies trifft auf die erfindungsgemäßen Peptidesequenzen bzw. Peptide zu.

**Peptide:**

Es wurde ein "Peptidscreening" mit ca. 450 ausgewählten unterschiedlichen Peptiden (15-22mere) bei dem oben beschriebenen Patientenkollektiv durchgeführt.

Die Peptide deckten das gesamte Virusprotein ab, wobei wir 15mere mit 5 Aminosäuren langen überlappenden Bereichen verwendeten um möglichst viele relevante Epitope zu erfassen.

Der Tabelle 1 sind die jeweiligen Positionen in HCV Genom der erfindungsgemäßen Epitope unter Angabe der jeweiligen Virusisolat Referenz zu entnehmen (Tabelle 1 / Spalte 4). Die Angaben zur Aminosäureposition (Tabelle 1 / Spalte 2) sind nur als Näherung zu verstehen, da aufgrund der hohen Mutationsrate des Virus bei den verschiedenen Virusisolaten zu Änderungen der Position kommen kann.

Aus früheren eigenen Untersuchungen ging hervor, daß bestimmten Bereichen des Virus besondere immunologische Bedeutung zukommt, deshalb wurden diese Bereiche des Virusgenoms (NS3-NS4) mit Hilfe von Peptiden (20mere mit 10 Aminosäuren langen überlappenden Bereichen) zusätzlich getestet.

In Tabelle 1 sind die erfindungsgemäßen Epitope aufgeführt, wobei deren jeweilge Impact Factoren (IF) angegeben sind.

Tabelle 1:

| Nr. | Pos | IF | Aminosäure Sequenz | Virusisolat Referenz (s.a. Liste 1) |
|---|---|---|---|---|
| EP001 | 20 | 16,0 | DVKFPGGGQIVGGVY | c,1,2,3,4,5,6,7,8,9,10,12,13,14,15,16 |
| EP002 | 40 | 24,0 | GPRLGVRATRKTSER | c,3,4,5,6,8,10,12,14,15,16 |
| EP003 | 60 | 18,0 | RRQPIPKARRPEGRA | c,1,7 |
| EP004 | 70 | 10,0 | PEGRAWAQPGYPWPL | c,1,7,9,15,16 |
| EP005 | 80 | 10,0 | YPWPLYGNEGLGWAG | c,2,4,7,9,15,16 |
| EP006 | 100 | 12,0 | RGSRPSWGPTDPRRR | c,2,3,6,7,8,10,16 |
| EP007 | 140 | 14,0 | GAPLGGAARALAHGV | c,1,2,3,6,7,8,10,15,16 |
| EP008 | 150 | 14,0 | LAHGVRVLEDGVNYA | c,1,2,3,6,7,8,9,10,15,16 |
| EP009 | 160 | 16,0 | GVNYATGNLPGCSFS | c,1,2,3,6,7,8,9,10,11,15,16 |
| EP010 | 190 | 12,0 | AYEVRNVSGVYHVTN | c,1 |
| EP011 | 200 | 14,0 | YHVTNDCSNSSIVYE | c,7,8,9,15,16 |
| EP012 | 210 | 12,0 | SIVYEAADMIMHTPG | c,8,9,15 |
| EP013 | 220 | 10,0 | MHTPGCVPCVRENNS | c,9 |
| EP014 | 300 | 9,6 | VQDCNCSIYPGHVSG | c,7,8,16 |
| EP015 | 340 | 10,0 | PQAVVDMVAGAHWGV | c,2,7,8,16 |
| EP016 | 360 | 12,0 | YYSMVGNWAKVLIVM | c,1,7,8,9,15 |
| EP017 | 380 | 9,6 | VDGGGGTTHVTGGAA | c |
| EP018 | 400 | 19,2 | GFTSLFSPGASQKIQ | c |
| EP019 | 420 | 10,0 | GSWHINRTALNCNDS | c,1,2,4,5,8,9,12,14,16 |
| EP020 | 430 | 14,0 | NCNDSLQTGFLAALF | c,2 |
| EP021 | 440 | 10,0 | LAALFYTHRFNSSGC | c |
| EP022 | 460 | 14,0 | SCRPIDKFAQGWGPI | c |
| EP023 | 550 | 10,0 | GNWFGCTWMNSTGFT | c,2,3,6,7,8,9,10,15,16 |

| EP024 | 560 | 16,0 | STGFTKTCGGPPCNI | c,2,7,8,9,15,16 |
|---|---|---|---|---|
| EP025 | 570 | 10,0 | PPCNIGGVGNNTLTC | c,1,2 |
| EP026 | 580 | 16,0 | NTLTCPTDCFRKHPE | c,1,2,15,16 |
| EP027 | 590 | 17,3 | RKHPEATYTKCGSGP | c,1,2,7,8,9,15,16 |
| EP028 | 600 | 14,0 | CGSGPWLTPRCLVDY | c,11,16 |
| EP029 | 690 | 11,5 | TGLIHLHQNIVDVQY | c,1,2,3,6,7,10,11,12,14,15,16 |
| EP030 | 850 | 14,0 | QYFITRAEAHLQVWV | c,8 |
| EP031 | 870 | 12,0 | RGGRDAIILLTCAVH | c,8,15,16 |
| EP032 | 910 | 12,0 | TRVPYFVRAQGLIRA | c,8,16 |
| EP033 | 920 | 15,4 | GLIRACMLVRKVAGG | c,1,6,8,16 |
| EP034 | 940 | 15,4 | AFMKLAALTGTYVYD | c,6,8 |
| EP035 | 980 | 12,0 | SDMETKIITWGADTA | c,1,2,6,9,15 |
| EP036 | 1000 | 9,3 | ILGLPVSARRGREIL | c,7,9,15,16 |
| EP037 | 1020 | 8,9 | SLEGQGWRLLAPITA | c,16 |
| EP038 | 1050 | 8,9 | LTGRDKNQVEGEVQV | c,1,2,7,9,16 |
| EP039 | 1060 | 12,5 | GEVQVVSTATQSFLA | c,1,2,7,9,16 |
| EP040 | 1100 | 10,7 | ITQMYTNVDQDLVGW | c,2,7,8,9 |
| EP041 | 1110 | 20,7 | DLVGWQAPPGARSLT | c |
| EP042 | 1120 | 27,6 | ARSLTPCTCGSSDLY | c,1,2,7,9 |
| EP043 | 1130 | 23,2 | SSDLYLVTRHADVIP | c,1,2,3,6,7,9,10,15,16 |
| EP044 | 1140 | 12,5 | ADVIPVRRRGDSRGS | c,1,2,3,6,9,10,15,16 |
| EP045 | 1160 | 13,8 | PVSYLKGSSGGPLLC | c,1,2,7,15,16 |
| EP046 | 1230 | 10,7 | HLHAPTGSGKSTKVP | c,1,2,3,6,7,8,9,10,12,13,15,16 |
| EP047 | 1240 | 12,5 | STKVPAAYAAQGYKV | c,1,2,3,6,7,8,9,13,15,16 |
| EP048 | 1250 | 33,9 | QGYKVLVLNPSVAAT | c,1,2,3,4,5,6,7,8,9,12,13,14,15,16 |
| EP049 | 1280 | 8,6 | NIRTGVRTITTGAPI | c,1,7,9 |
| EP050 | 1290 | 17,2 | TGAPITYSTYGKFLA | 1,4,7,9,12,15 |
| EP051 | 1360 | 8,9 | VPHPNIEEVALSNTG | c,2,7,8 |
| EP052 | 1370 | 16,1 | LSNTGEIPFYGKAIP | c,2,7,8,9 |
| EP053 | 1379 | 11,5 | AIPLEVIKGGRHLIFCHSKR | 17 |
| EP054 | 1380 | 10,7 | GKAIPIEVIKGGRHL | c |
| EP055 | 1390 | 23,2 | GGRHLIFCHSKKKCD | c,1,4,7,9,12,15 |
| EP056 | 1400 | 17,9 | KKKCDELAAKLSALG | c,9,15 |
| EP057 | 1406 | 12,5 | KLVAMGINAVAYYRGLDVSV | 17 |
| EP058 | 1410 | 12,5 | LSALGLNAVAYYRGL | c |
| EP059 | 1420 | 12,5 | YYRGLDVSVIPTSGD | c,1,3,6,7,8,10,12,15 |
| EP060 | 1424 | 10,6 | SVIPTSGDVVVVATDALMTG | c,1,6,8,15,17 |
| EP061 | 1470 | 13,8 | DPTFTIETTTVPQDA | c,1,2,7,13,16 |
| EP062 | 1500 | 10,7 | GIYRFVTPGERPSGM | c,1,2,7,8,9,11,15,16 |
| EP063 | 1510 | 8,9 | RPSGMFDSSVLCECY | c,1,2,3,6,7,8,9,10,15,16 |
| EP064 | 1535 | 14,7 | TTVRLRAYMNTPGLPVCQ | 3,6,10,14 |
| EP065 | 1540 | 19,6 | VRLRAYLNTPGLPVC | c,1,2,7,8,9,13,15,16 |
| EP066 | 1575 | 11,8 | QTKQSGENLPYLVAYQATVC | 6 |
| EP067 | 1585 | 17,6 | YLVAYQATVCARAQAPPPSW | c,2,3,6,7,8,9,10,15,16 |
| EP068 | 1595 | 7,8 | ARAQAPPPSWDQMWKCLIRL | c,1,2,3,6,7,8,15,16 |
| EP069 | 1600 | 12,5 | AQAPPPSWDQMWKCL | c,1,2,3,6,7,8,9,15,16 |
| EP070 | 1605 | 8,8 | DQMWKCLIRLKPTLHGPTPL | c,1,2,3,6,7,8,15,16 |
| EP071 | 1610 | 24,1 | MWKCLIRLKPTLHGP | c,1,2,3,6,7,8,12,15,16 |
| EP072 | 1660 | 9,3 | TSTWVLVGGVLAALA | c,1,2,3,6,7,8,9,10,12,14,15,16 |

| EP073 | 1690 | 17,9 | ILSGRPAVIPDREVL | c,8 |
|---|---|---|---|---|
| EP074 | 1720 | 9,3 | YIEQGMQLAEQFKQK | c,1,2,7,8,9,15,16 |
| EP075 | 1750 | 16,7 | APVVESKWRALETFW | c,2,16 |
| EP076 | 1755 | 10,0 | QKLETFWAKHMWNFISGIQY | 6 |
| EP077 | 1765 | 12,0 | MWNFISGIQYLAGLSTLPGN | c,1,2,3,4,5,6,7,8,9,10,12,15,16 |
| EP078 | 1770 | 13,0 | NFISGIQYLAGLSTL | c,1,2,3,4,5,6,7,8,9,10,12,15,16 |
| EP079 | 1775 | 11,0 | LAGLSTLPGNPAIASLMAFT | c,2,3,6,7,8,9,10,16 |
| EP080 | 1780 | 9,3 | GLSTLPGNPAIASLM | c,2,3,6,7,8,9,10,12,16 |
| EP081 | 1805 | 10,0 | QTLLFNILGGWVAAQLAAPG | 3,6,10 |
| EP082 | 1810 | 40,7 | LLFNILGGWVAAQLA | c,1,2,3,6,7,8,9,10,15,16 |
| EP083 | 1820 | 11,1 | AAQLAPPSAASAFVG | c,1,2,7,8,15,16 |
| EP084 | 1830 | 12,5 | SAFVGAGIAGAAVGS | c,1,2,8,9,15,16 |
| EP085 | 1850 | 24,1 | VLVDILAGYGAGVAG | c,1,2,7,8,10,15,16 |
| EP086 | 1870 | 11,1 | KVMSGEMPSTEDLVN | c,1,2,8,9 |
| EP087 | 1880 | 19,6 | EDLVNLLPAILSPGA | c,1,2,3,6,7,8,9,10,12,15,16 |
| EP088 | 1935 | 26,7 | THYVPESDAAARVTQILSSL | c,1,2,7,8,16 |
| EP089 | 1955 | 43,8 | TITQLLKRLHQWINEDCSTP | c,1,2,7,8,16 |
| EP090 | 1960 | 9,3 | LKRLHQWINEDCSTP | c,1,2,7,8,16 |
| EP091 | 1975 | 23,3 | CSGSWLRDVWDWICTVLTDF | c,1,2,15,16 |
| EP092 | 1980 | 9,3 | LRDVWDWICTVLTDF | c,1,2,7,15,16 |
| EP093 | 1990 | 9,3 | VLTDFKTWLQSKLLP | c,1,2,7,15,16 |
| EP094 | 2000 | 9,3 | SKLLPRLPGVPFFSC | c,9 |
| EP095 | 2020 | 14,0 | GVWRGDGIMQTTCPC | c,1,2,7,8,9,16 |
| EP096 | 2025 | 16,7 | DGIMQTTCPCGAQITGHVKN | c,1,2,7,8 |
| EP097 | 2030 | 10,0 | TTCPCGAQITGHVKN | c,1,2,7,8,15 |
| EP098 | 2035 | 23,3 | GAQITGHVKNGSMRIVGPKT | c,1,2,7,8,15 |
| EP099 | 2080 | 10,0 | NYSRALWRVAAEEYV | c,1,2,7,8,9,15,16 |
| EP100 | 2085 | 20,0 | LWRVAAEEYVEVTRVGDFHY | c,1,2,8,15,16 |
| EP101 | 2090 | 10,0 | AEEYVEVTRVGDFHY | c,1,2,8,15,16 |
| EP102 | 2095 | 21,9 | EVTRVGDFHYVTGMTTDNVK | c,1,2,8,15,16 |
| EP103 | 2115 | 23,3 | CPCQVPAPEFFTEVDGVRLH | c,1,8,9,15,16 |
| EP104 | 2120 | 12,0 | PAPEFFTEVDGVRLH | c,1,8,9,15,16 |
| EP105 | 2125 | 28,1 | FTEVDGVRLHRYAPACKPLL | c,1,9,15,16 |
| EP106 | 2130 | 11,5 | GVRLHRYAPACKPLL | c,1,9,15,16 |
| EP107 | 2145 | 16,7 | REEVTFQVGLNQYLVGSQLP | c,2 |
| EP108 | 2150 | 10,0 | FQVGLNQYLVGSQLP | c,2,7,8,16 |
| EP109 | 2175 | 26,7 | TSMLTDPSHITAETAKRRLA | c,2,7,8,9,15,16 |
| EP110 | 2180 | 10,0 | DPSHITAETAKRRLA | c,2,7,8,9,15,16 |
| EP111 | 2190 | 10,0 | KRRLARGSGVPPSLA | c |
| EP112 | 2200 | 10,0 | PPSLASSSASQLSAP | c,1,2,7,8,15,16 |
| EP113 | 2205 | 23,3 | SSSASQLSAPSLKATCTTHH | c,2,7,8,16 |
| EP114 | 2210 | 10,0 | QLSAPSLKATCTTHH | c,2,7,8,16 |
| EP115 | 2215 | 16,7 | SLKATCTTHHSYNLDSPDAD | c |
| EP116 | 2260 | 10,0 | VILDSFDPLRAEEDE | c,1,7,9 |
| EP117 | 2265 | 20,0 | FDPLRAEEDEREVSVAAEIL | c,1,7 |
| EP118 | 2275 | 25,0 | REVSVAAEILRKSRKFPPAM | c |
| EP119 | 2295 | 16,7 | PIWARPDYNPPLLESWKDPD | c,1,2,8,16 |
| EP120 | 2305 | 26,7 | PLLESWKDPDYVPPVVHGCP | c,1,2,15,16 |
| EP121 | 2315 | 16,7 | YVPPVVHGCPLPPTKAPPIP | c,1,16 |

| EP122 | 2380 | 10,0 | QASDDGDKGSDVESY | c,2,8 |
|---|---|---|---|---|
| EP123 | 2425 | 23,3 | DVVCCSMSYTWTGALITPCA | c,1,2,8,16 |
| EP124 | 2435 | 20,0 | WTGALITPCAAEESKLPINA | c,1,2,7,9 |
| EP125 | 2560 | 10,0 | ETPIDTTIMAKNEVF | c,1,8,9,15 |
| EP126 | 2570 | 12,0 | KNEVFCVQPEKGGRK | c,1,2,3,6,8,9,10,15 |
| EP127 | 2580 | 10,0 | KGGRKPARLIVFPDL | c,1,2,3,6,7,8,9,10,16 |
| EP128 | 2610 | 12,0 | TLPQAVMGSSYGFQY | c,7,9,15,16 |
| EP129 | 2650 | 10,0 | TRCFDSTVTENDIRV | c,2 |
| EP130 | 2670 | 12,0 | QCCDLAPEARQAIRS | c,8,9,16 |
| EP131 | 2690 | 10,0 | YIGGPLTNSKGQNCG | c,1,2,7,9,15 |
| EP132 | 2700 | 18,0 | GQNCGYRRCRASGVL | c,1,2,7,8,9,15,16 |
| EP133 | 2810 | 10,0 | VYYLTRDPTTPLARA | c,1,2,3,6,7,8,9,10,15,16 |
| EP134 | 2890 | 12,0 | QIIQRLHGLSAFSLH | 1,7,15 |
| EP135 | 2900 | 10,0 | AFSLHSYSPGEINRV | c,1,2,3,6,7,8,9,10,15 |
| EP136 | 2910 | 18,0 | EINRVASCLRKLGVP | c,1,2,7,8,9,13,15,16 |
| EP137 | 2920 | 10,0 | KLGVPPLRVWRHRAR | c,2,7,8,15 |
| EP138 | 2950 | 12,0 | CGKYLFNWAVRTKLK | c,8,9,16 |
| EP139 | 2960 | 10,0 | RTKLKLTPIPAASQL | c,7,15,16 |
| EP140 | 3010 | 16,7 | VGVGIYLLPNR | c,7,8,9,15,16 |

**Virusisolate**

**1**
Genotype: 1b AUTHORS Trowbridge,R. and Gowans,E.J.
TITLE Molecular cloning of an Australian isolate of hepatitis C virus
JOURNAL Arch. Virol. 143 (3), 501-511 (1998)
**2**
Genotype: 1b AUTHORS Takamizawa,A., Mori,C., Fuke,I., Manabe,S., Murakami,S., Fujita,J., Onishi,E., Andoh,
T., Yoshida,I. and Okayama,H.
TITLE Structure and organization of the hepatitis C virus genome isolated from human carriers
JOURNAL J. Virol. 65 (3), 1105-1113 (1991)
**3**
Genotype: 1a AUTHORS Yanagi,M., Purcell,R.H., Emerson,S.U. and Bukh,J.
TITLE Transcripts from a single full-length cDNA clone of hepatitis C virus are infectious when directly transfected
into the liver of a chimpanzee
JOURNAL Proc. Natl. Acad. Sci. U.S.A. 94 (16), 8738-8743 (1997)
**4**
Genotype:2a AUTHORS Okamoto,H., Okada,S., Sugiyama,Y., Kurai,K., Iizuka,H., Machida,A., Miyakawa,Y. and
Mayumi,M.
TITLE Nucleotide sequence of the genomic RNA of hepatitis C virus isolated from a human JOURNAL J. Gen.
Virol. 72 (Pt 11), 2697-2704 (1991)
**5**
Genotype:2b AUTHORS Okamoto,H., Kurai,K., Okada,S., Yamamoto,K., Lizuka,H., Tanaka,T., Fukuda,S., Tsuda,
F. and Mishiro,S.
TITLE Full-length sequence of a hepatitis C virus genome having poor homology to reported isolates: comparative
study of four distinct genotypes
JOURNAL Virology 188 (1), 331-341 (1992)
**6**
Genotype: 1a AUTHORS CHOO,Q.-L., RICHMAN,K.H., HAN,J.H., BERGER,K., LEE,C., DONG,C., GALLEGOS,
C, COIT,D., MEDINA-SELBY,A., BARR,P.J., WEINER,A.J., BRADLEY,D.W., KUO,G. and HOUGHTON,M.

TITLE Genetic organization and diversity of the hepatitis C virus
JOURNAL Proc. Natl. Acad. Sci. U.S.A. 88 (6), 2451-2455 (1991)

**7**

Genotype: 1b AUTHORS Tanaka,T., Kato,N., Nakagawa,M., Ootsuyama,Y., Cho,M.J., Nakazawa,T., Hijikata,M., Ishimura,Y. and Shimotohno,K.
TITLE Molecular cloning of hepatitis C virus genome from a single Japanese carrier: sequence variation within the same individual and among infected individuals
JOURNAL Virus Res. 23 (1-2), 39-53 (1992

**8**

Genotype:1b AUTHORS Kato,N., Hijikata,M., Ootsuyama,Y., Nakagawa,M., Ohkoshi,S.,Sugimura,T. and Shimotohno,K.
TITLE Molecular cloning of the human hepatitis C virus genome from Japanese patients with non-A, non-B hepatitis
JOURNAL Proc. Natl. Acad. Sci. U.S.A. 87 (24), 9524-9528 (1990)

**9**

Genotype:1b AUTHORS Chen,P.J., Lin,M.H., Tai,K.F., Liu,P.C., Lin,C.J. and Chen,D.S.
TITLE The Taiwanese hepatitis C virus genome: sequence determination and mapping the 5' termini of viral genomic and antigenomic RNA
JOURNAL Virology 188 (1), 102-113 (1992)

**10**

Genotype:1a AUTHORS Inchauspe,G., Zebedee,S., Lee,D.H., Sugitani,M., Nasoff,M. and Prince,A.M.
TITLE Genomic structure of the human prototype strain H of hepatitis C virus: comparison with American and Japanese isolates
JOURNAL Proc. Natl. Acad. Sci. U.S.A. 88 (22), 10292-10296 (1991)

**11**

Genotype:3b AUTHORS Chayama,K. Toranomon Hospital, Department of Gastroenteroloty; 2-2-2 Toranomon, Minato-ku, Tokyo 105, Japan
TITLE Direct Submission
JOURNAL Submitted (18-FEB-1995) to the DDBJ/EMBL/GenBank databases. COMMENT D26556:Submitted (20-Jan-1994) to DDBJ by: Kazuaki Chayama.

**12**

Genotype:4 AUTHORS ::Chamberlain RW, Adams N, Saeed AA, Simmonds P, Elliott RM
TITLE : Complete nucleotide sequence of a type 4 hepatitis C virus variant, the predominant genotype in the Middle East.
JOURNAL: J Gen Virol. 1997 Jun;78 ( Pt 6):1341-7.

**13**

Genotype:5a AUTHORS Chamberlain RW, Adams NJ, Taylor LA, Simmonds P, Elliott RM.
TITLE The complete coding sequence of hepatitis C virus genotype 5a, the predominant genotype in South Africa.
JOURNAL Biochem Biophys Res Commun. 1997 Jul 9;236(1):44-9.

**14**

Genotype: 6a AUTHORS Adams, N.J., Chamberlain,R.W., Taylor,L.A., Davidson,F., Lin,C.K.,Elliott,R.M. and Simmonds,P.
TITLE Complete coding sequence of hepatitis C virus genotype 6a
JOURNAL Biochem. Biophys. Res. Commun. 234 (2), 393-396 (1997)

**15**

Genotype:1b AUTHORS Honda,M., Kaneko,S., Unoura,M., Kobayashi,K. and Murakami,S.
TITLE Sequence comparisons for a hepatitis C virus genome RNA isolated from a patient with liver cirrhosis
JOURNAL Gene 120 (2), 317-318 (1992)

**16**

Genotype:1b AUTHORS Okamoto,H., Kojima,M., Okada,S., Yoshizawa,H., Iizuka.H., Tanaka,T., Muchmore,E.E., Peterson,D.A., Ito,Y. and Mishiro,S.
TITLE Genetic drift of hepatitis C virus during an 8.2-year infection in a chimpanzee: variability and stability
JOURNAL Virology 190 (2), 894-899 (1992)

**17**

Genotype:1a AUTHORS: Choo QL, Kuo G, Weiner AJ, Overby LR, Bradley DW, Houghton M.
Title: Isolation of a cDNA clone derived from a blood-borne non-A, non-B viral hepatitis genome.
Journal: Science 1989 Apr 21;244(4902):359-62

**C**

Genotype:1b

Primary consensus sequence complete 1b genomes available in EMBL database (January 2000)

Die erfindungsgemäßen Epitope zeichnet weiterhin aus, daß eine deutliche spezifische CD4+ T-Zell Aktivität auf diese Peptide mit einer Abnahme des Virustiters korreliert. Gerade diese Epitope dürften somit ideale Kandidaten für eine Vakzine darstellen. Eine spezifisch Impfreaktion gegen diese Peptide, könnte die Erkrankung auf der einen Seite verhindern und/oder auf der anderen Seite zu deren Ausheilung führen, zumindest aber den Verlauf einer HCV Infektion günstig beeinflussen.

Die erfindungsgemäßen Epitope sind hochimmunogene, hochkonservierte und zum Teil in unmittelbarer Nachbarschaft zu bekannten CD8+ T-Lymphozyten spezifischen HCV-Epitopen gelegene Sequenzen des HCV. Als gegenüber CD4$^+$ T-Lymphozyten spezifische HCV-Epitope können diese neben der Induktion von CD4+ T-Lymphozyten auch sogenannte T-Zell-Hilfe für zytotoxische CD8+ T-Lymphozyten vermitteln. Diese CD8+-T-Lymphozyten werden durch die Zytokine stimulierter CD4+ T-Lymphozyten aktiviert.

Des weiteren zeichnen sich die Peptide durch häufige signifikante Reaktion bei verschiedenen Patienten mit unterschiedlichen MHC Klasse II Typen (major histocompability complex) aus. Das MHC Klasse II System ist ausgesprochen polymorph. Die Aufgabe der MHC Moleküle ist es von körpereigenen und pathogenen (z.B. Hepatitis C Virus) Proteinen stammende Peptidfragmente zu binden und sie zur Erkennung und Aktivierung von spezifischen CD4+T-Lymphozyten an der Zelloberfläche zu exprimieren. Dieses System ermöglicht eine wirkungsvolle und spezifische Immunantwort gegen Pathogene wie z.B. HCV. Da nun verschiedene MHC Klasse II Typen, d.h. unterschiedliche Personen, dasselbe Peptid auf ihrem MHC Klasse II Molekül exprimieren können, was in vitro durch bei unterschiedlichen Personen meßbare, gegen dasselbe Peptid gerichtete CD4+ Aktivität wiederfindet, kann bei diesen Peptiden von einer Promiskuität ausgegangen werden. Dies bedeutet, daß die erfindungsgemäßen Epitope immunologische Relevanz bei verschiedenen Individuen besitzen.

Zusammenfassend eignen sich gerade die erfindungsgemäßen Epitope im hohen Maße sowohl für eine therapeutische als auch prophylaktische Peptidimpfung, die gegen das HCV gerichtet ist.

**Testsystem:**

Als Testsystem wurde der sogenannte Proliferationsassay nach folgendem Protokoll eingesetzt. Nach Dichtegradientenzentrifugation auf Ficollgradienten von heparinisiertem Blut wurden die frischen peripheren mononukleären Blutzellen (PBMC) isoliert und in einem Kulturmedium (RPMI1640, Gibco) suspendiert. 50 μl dieser Zellsuspension (Konzentration von 1x10$^6$ Zellen pro ml) wurden auf sterile 96 Loch-Kulturplatten verbracht. Durch Zugabe der Peptide wurden die Zellen stimuliert. Die Endkonzentration der Peptide betrug 10μg/ml. Die Zellkulturplatten wurden über 5 Tage bei 37°C und 5%CO$_2$ kultiviert, anschließend mit $^3$H-Thymidin versetzt und als Maß für die Zellstimulation wurde der Einbau des radioaktiven $^3$H gemessen.

**Auswertung:**

Bei unseren Untersuchungen wurden nur Peptidreaktionen als signifikant angesehen, deren Stimulationsindex (SI) größer 3 war (3x erhöht gegenüber den Kontrollen, bzw. den irrelevanten Peptiden).

Um einerseits die Möglichkeit falsch positiver Reaktionen sowie irrelevanter Kreuzreaktionen zu minimieren und andererseits eine Hierarchie hinsichtlich der biologischen Wertigkeit relevanter Epitope zu erstellen, wurde ein zusätzlicher Filter definiert und im Folgenden als Impact factor (IF) bezeichnet.

Dem Impact factor (IF) liegt ein Punktesystem zugrunde, welches nicht nur die Häufigkeit relevanter Reaktionen, d.h. Stimulationsindex (SI) größer 3, wie herkömmlicherweise, verwendet, sondern auch die Stärke dieser Reaktionen berücksichtigt.

Dieses Bewertungssystem wurde für jedes untersuchte Peptid angewendet und ist nach folgender Formel definiert:

$$IF = \frac{n_1 * 1 + n_2 * 1{,}5}{m} * 100 \qquad \text{(Formel 1)}$$

wobei

$n_1$ der Summe der Reaktionen mit $3 < SI < 6$,

$n_2$ der Summe der Reaktionen mit $SI \geq 6$ und

m der Anzahl der Tests gegen das jeweilige Peptid entspricht, wobei $m \geq 15$ sein muß.

Beispiel:

Gegen EP82 wurden in 27 (m) unabhängigen Versuchen folgende spezifischen SI's (Stimulationsindex) gemessen: 0,60; 1,66; 0,78; 1,18; 0,84; 1,65; 0,24; 1,33; 0,94; 2,34; 20,07; 0,68; 1,43; 33,35; 10,27; 0,83; 0,98; 0,82; 1,40; 0,80; 3,38; 3,48; 2,15; 0,44; 13,16; 10,83; 11,11.

Damit ergibt sich nach Formel 1 einen Impact factor von 40,74 für dieses Peptid. Da bei den von uns getesteten Peptiden der MW aller Impact factoren 7,36 und die Standardabweichung 6,75 betrug, gehört dieses Epitop zur Kategorie I entsprechend einem Impact factor von 40,74 und damit ≥ MW +2*Sta. Für jedes untersuchte Peptid wurde der jeweilige Impact factor berechnet. Aus allen Impact factoren ließen sich dann Mittelwert und Standardabweichung errechnen.

Somit ergibt sich je nach Höhe des Impact factors eine biologischimmunologisch bedeutsame Hierarchie. Peptide mit hohem Impact factor zeichnen sich nicht nur durch einen großen Stimulationsindex, d.h. starke spezifische Reaktiviät, sondern auch durch die wiederholt d.h. bei unterschiedlichen Personen gefundene spezifische Reaktion aus.

Um qualitative, immunologisch bedeutsame Unterschiede herauszustellen, wurden die von uns gefundenen Peptide, bzw. Epitope in 3 hierarchische Kategorien eingeteilt. Nach der Berechnung des individuellen Impact factors für jedes Peptid, erfolgte die Kategorisierung in drei Gruppen. Hierzu wurde der arithmetische Mittelwert (MW) und die Standardabweichung (Sta) aller Impact factors errechnet und die folgenden Kategorien gebildet:

Kategorie I: Impact factor ≥ MW +2*Sta
Kategorie II: Impact factor > MW +1*Sta und < MW+2*Sta
Kategorie III: Impact factor > MW und ≤ MW+1*Sta

Diese hier getroffene Selektion soll der immunologischen Wertigkeit, der hier aufgeführten Peptide, besser gerecht werden. Epitope, die starke und bei unterschiedlichen Patienten gemessene HCV spezifisch CD4+ T-Zell Antworten auslösen, haben für künftige Impfansätze eine hohe Relevanz.

Insbesondere bevorzugt werden die erfindungsgemäßen Epitope enthalten in den nachfolgend aufgeführten Sequenzen ausgewählt aus der Gruppe, bestehend aus:

| | |
|---|---|
| EP002 | GPRLGVRATRKTSER |
| EP042 | ARSLTPCTCGSSDLY |
| EP043 | SSDLYLVTRHADVIP |
| EP048 | QGYKVLVLNPSVAAT |
| EP055 | GGRHLIFCHSKKKCD |
| EP071 | MWKCLIRLKPTLHGP |
| EP082 | LLFNILGGWVAAQLA |
| EP085 | VLVDILAGYGAGVAG |
| EP088 | THYVPESDAAARVTQILSSL |
| EP089 | TITQLLKRLHQWINEDCSTP |
| EP091 | CSGSWLRDVWDWICTVLTDF |
| EP098 | GAQITGHVKNGSMRIVGPKT |
| EP102 | EVTRVGDFHYVTGMTTDNVK |
| EP103 | CPCQVPAPEFFTEVDGVRLH |
| EP105 | FTEVDGVRLHRYAPACKPLL□ |
| EP109 | TSMLTDPSHITAETAKRRLA |
| EP113 | SSSASQLSAPSLKATCTTHH |
| EP118 | REVSVAAEILRKSRKFPPAM |
| EP120 | PLLESWKDPDYVPPVVHGCP |
| EP123 | DVVCCSMSYTWTGALITPCA |

Diese bevorzugten Sequenzen umfassen 20 Peptide, bzw. Epitope mit einem Impact factor ≥ MW +2*Sta (=Kategorie I).
Des Weiteren werden die erfindungsgemäßen Epitope enthalten in den nachfolgend aufgeführten Sequenzen bevorzugt ausgewählt aus der Gruppe, bestehend aus:

| | |
|---|---|
| EP001 | DVKFPGGGQIVGGVY |
| EP003 | RRQPIPKARRPEGRA |
| EP009 | GVNYATGNLPGCSFS |

| EP018 | GFTSLFSPGASQKIQ |
|-------|-----------------|
| EP024 | STGFTKTCGGPPCNI |
| EP026 | NTLTCPTDCFRKHPE |
| EP027 | RKHPEATYTKCGSGP |
| EP033 | GLIRACMLVRKVAGG |
| EP034 | AFMKLAALTGTYVYD |
| EP041 | DLVGWQAPPGARSLT |
| EP050 | TGAPITYSTYGKFLA |
| EP052 | LSNTGEIPFYGKAIP |
| EP056 | KKKCDELAAKLSALG |
| EP064 | TTVRLRAYMNTPGLPVCQ |
| EP065 | VRLRAYLNTPGLPVC |
| EP067 | YLVAYQATVCARAQAPPPSW |
| EP073 | ILSGRPAVIPDREVL |
| EP075 | APVVESKWRALETFW |
| EP087 | EDLVNLLPAILSPGA |
| EP096 | DGIMQTTCPCGAQITGHVKN |
| EP100 | LWRVAAEEYVEVTRVGDFHY |
| EP107 | REEVTFQVGLNQYLVGSQLP |
| EP115 | SLKATCTTHHSYNLDSPDAD |
| EP117 | FDPLRAEEDEREVSVAAEIL |
| EP119 | PIWARPDYNPPLLESWKDPD |
| EP121 | YVPPVVHGCPLPPTKAPPIP |
| EP124 | WTGALITPCAAEESKLPINA |
| EP132 | GQNCGYRRCRASGVL |
| EP136 | EINRVASCLRKLGVP |
| EP140 | VGVGIYLLPNR |

Diese Gruppe an Sequenzen umfaßt 30 Peptide, bzw. Epitope mit einem Impact factor > MW +1*Sta und < MW+2*Sta (= Kategorie II).

Des Weiteren werden die erfindungsgemäßen Epitope enthalten in den nachfolgend aufgeführten Sequenzen ausgewählt aus der Gruppe, bestehend aus:

EP004  PEGRAWAQPGYPWPL

EP005  YPWPLYGNEGLGWAG

EP006  RGSRPSWGPTDPRRR

EP007  GAPLGGAARALAHGV

EP008  LAHGVRVLEDGVNYA

EP010  AYEVRNVSGVYHVTN

EP011  YHVTNDCSNSSIVYE

EP012  SIVYEAADMIMHTPG

EP013  MHTPGCVPCVRENNS

EP014  VQDCNCSIYPGHVSG

EP015  PQAVVDMVAGAHWGV

EP016  YYSMVGNWAKVLIVM

EP017  VDGGGGTTHVTGGAA

EP019  GSWHINRTALNCNDS

EP020  NCNDSLQTGFLAALF

EP021  LAALFYTHRFNSSGC

EP022  SCRPIDKFAQGWGPI

EP023  GNWFGCTWMNSTGFT

EP025  PPCNIGGVGNNTLTC

EP028  CGSGPWLTPRCLVDY

EP029  TGLIHLHQNIVDVQY

EP030  QYFITRAEAHLQVWV

EP031  RGGRDAIILLTCAVH

EP032  TRVPYFVRAQGLIRA

EP035  SDMETKIITWGADTA

EP036  ILGLPVSARRGREIL

EP037  SLEGQGWRLLAPITA

EP038  LTGRDKNQVEGEVQV

EP039  GEVQVVSTATQSFLA

EP040  ITQMYTNVDQDLVGW

EP044  ADVIPVRRRGDSRGS

EP045  PVSYLKGSSGGPLLC

EP046  HLHAPTGSGKSTKVP

EP047  STKVPAAYAAQGYKV

EP049  NIRTGVRTITTGAPI

EP051  VPHPNIEEVALSNTG

EP053  AIPLEVIKGGRHLIFCHSKR

EP054  GKAIPIEVIKGGRHL

EP057  KLVAMGINAVAYYRGLDVSV

EP058  LSALGLNAVAYYRGL

EP059  YYRGLDVSVIPTSGD

EP060  SVIPTSGDVVVVATDALMTG

EP061  DPTFTIETTTVPQDA

EP062  GIYRFVTPGERPSGM

EP063  RPSGMFDSSVLCECY

EP066  QTKQSGENLPYLVAYQATVC

EP068  ARAQAPPPSWDQMWKCLIRL

EP069  AQAPPPSWDQMWKCL

EP070  DQMWKCLIRLKPTLHGPTPL

EP072  TSTWVLVGGVLAALA

EP074  YIEQGMQLAEQFKQK

EP076  QKLETFWAKHMWNFISGIQY

EP077  MWNFISGIQYLAGLSTLPGN

EP078  NFISGIQYLAGLSTL

EP079  LAGLSTLPGNPAIASLMAFT

EP080  GLSTLPGNPAIASLM

EP081  QTLLFNILGGWVAAQLAAPG

EP083  AAQLAPPSAASAFVG

EP084  SAFVGAGIAGAAVGS

EP086  KVMSGEMPSTEDLVN

EP090   LKRLHQWINEDCSTP

EP092   LRDVWDWICTVLTDF

EP093   VLTDFKTWLQSKLLP

EP094   SKLLPRLPGVPFFSC

EP095   GVWRGDGIMQTTCPC

EP097   TTCPCGAQITGHVKN

EP099   NYSRALWRVAAEEYV

EP101   AEEYVEVTRVGDFHY

EP104   PAPEFFTEVDGVRLH

EP106   GVRLHRYAPACKPLL

EP108   FQVGLNQYLVGSQLP

EP110   DPSHITAETAKRRLA

EP111   KRRLARGSGVPPSLA

EP112   PPSLASSSASQLSAP

EP114   QLSAPSLKATCTTHH

EP116   VILDSFDPLRAEEDE

EP122   QASDDGDKGSDVESY

EP125   ETPIDTTIMAKNEVF

EP126   KNEVFCVQPEKGGRK

EP127   KGGRKPARLIVFPDL

EP128   TLPQAVMGSSYGFQY

EP129   TRCFDSTVTENDIRV

EP130   QCCDLAPEARQAIRS

EP131   YIGGPLTNSKGQNCG

EP133   VYYLTRDPTTPLARA

EP134   QIIQRLHGLSAFSLH

EP135   AFSLHSYSPGEINRV

EP137   KLGVPPLRVWRHRAR

EP138   CGKYLFNWAVRTKLK

EP139   RTKLKLTPIPAASQL

Diese Gruppe umfaßt 90 Sequenzen, bzw. Epitope, Impact factor > MW und ≤ MW+1*Sta (= Kategorie III).

Eine weitere Lösung ist ein Impfstoff, der eine Kombination der erfindungsgemäßen Epitope EP1) bis EP140) enthält. Bevorzugt umfaßt der Impfstoff ein oder mehrere Epitope ausgewählt aus der Gruppe bestehend aus Epitopen der Kategorie I. Der Impfstoff kann insbesondere bevorzugt ein Gemisch aus den erfindungsgemäßen Epitopen EP1) bis EP140) enthalten. Allerdings können auch weitere HCV Epitope anwesend sein.

Die erfindungsgemäßen Epitope können allein oder mit einem oder mehreren Hilfsstoffen als Arzneimittel, vorzugsweise als Impfstoff, eingesetzt werden. Der erfindungsgemäße Impfstoff enthält mindestens ein erfindungsgemäßes Epitop, vorzugsweise ein Gemisch aus erfindungsgemäßen Epitopen, vorzugsweise ein Gemisch von erfindungsgemäßen Epitopen der Kategorie I. Allerdings können auch weitere HCV Epitope anwesend sein.

Die Hilfsstoffe werden vorzugsweise ausgewählt aus der Gruppe, bestehend aus fowlpoxvirus, modifiziertes Vakziniavirus Ankara, Virosomen, Transvax® und anderen die Immunreaktion verstärkenden Stoffen.

Der erfindungsgemäße Impfstoff kann oral, parenteral, intramuskulär, intravenös, subcutan oder intracutan verabreicht werden.

Bei den erfindungsgemäßen Epitopen handelt es sich um Epitope, die als T-Zell-stimulierender Impfstoff eingesetzt werden können. Ein Impfstoff, der die erfindungsgemäßen Epitope enthält, hat gegenüber einer Impfung mit dem gesamten Virusprotein, das verschiedenste Epitope für virusspezifische T-Lymphozyten enthält und nur B-Lymphozyten und CD4$^+$T-Lymphozyten induziert, den Vorteil, dass es spezifische T-Lymphozyten, CD4$^+$- und/oder CD8$^+$-T-Lymphozyten selektiv induziert.

Außerdem werden dadurch antagonistische Effekte bzw. die Gefahr von iatrogen erzeugten Autoimmunreaktionen, die bei Impfungen mit ganzen Proteinen auftreten können, vermieden. Die erfindungsgemäßen Epitope haben zusätzlich eine höhere Immunogenität im Vergleich zu dem gesamten Virusprotein, wodurch ein besseres Impfergebnis erreicht wird.

Der erfindungsgemäße Impfstoff ermöglicht somit im gesunden Menschen die Induktion einer Immunantwort und dient daher als prophylaktische Impfung. Auch bei chronisch HCV-infizierten Menschen kann der erfindungsgemäße Impfstoff eine Immunantwort induzieren und somit als therapeutischer Impfstoff dienen.

Die kodierende cDNA dieser Epitope kann in einem DNA-Impfstoff, einer speziellen Impfmethode, eingesetzt werden. Dabei wird die für die entsprechenden Epitope codierende DNA in einen Vector kloniert. Dieses Konstrukt wird wiederum dem zu impfenden Individuum parenteral verabreicht (z.B. Immunology and Cell Biology, Band 75, Seite 382 bis 388). Entsprechend des degenerierten genetischen Codes können verschiedene DNA-Sequenzen eines der erfindungsgemäßen Epitope kodieren (siehe Current protocols, Wiley).

Die erfindungsgemäßen Epitope können auch in der Diagnose des Verlaufs einer HCV-Infektion Verwendung finden, indem die Menge an CD4$^+$ T-Lymphozyten, die spezifisch das betreffende Epitop erkennen, im Blut des Patienten mit einer Hepatitis C Infektion überwacht wird. Dies kann beispielsweise mit einem diagnostischen Kit durchgeführt werden, der eines oder mehrere der erfindungsgemäßen Epitope umfasst.

**Patentansprüche**

1. CD4$^+$ T-Lymphozyten spezifische HCV-Epitope
   mit einem Impact factor (IF) > Mittelwert (MW)
   wobei

$$IF = \frac{n_1 *1 + n_2 *1,5}{m}*100 \qquad \text{(Formel 1)}$$

   wobei
   $n_1$ der Summe der Reaktionen mit 3 < SI < 6,
   $n_2$ der Summe der Reaktionen mit SI $\geq$ 6 und
   m der Anzahl der Tests gegen das jeweilige Peptid entspricht, wobei m $\geq$15 ist, und MW der Mittelwert aller Impact factoren ist.

2. CD4$^+$ T-Lymphozyten spezifische HCV-Epitope nach Anspruch 1, mit
   zumindest zweimaliger signifikanter Reaktion, nämlicheinem SI > 3, bei einer Testanzahl m $\geq$ 15.

3. CD4$^+$ T-Lymphozyten spezifische HCV-Epitope nach Anspruch 1 oder 2 mit einem Impact factor > MW und $\leq$ MW+1*Sta.

4. CD4$^+$ T-Lymphozyten spezifische HCV-Epitope nach Anspruch 1 oder 2 mit einem Impact factor > MW +1*Sta und < MW+2*Sta.

5. CD4$^+$ T-Lymphozyten spezifische HCV-Epitope nach Anspruch 1 oder 2 mit einem Impact factor $\geq$ MW +2*Sta.

6. CD4$^+$ T-Lymphozyten spezifische HCV-Epitope nach Anspruch 3, umfassend ein oder mehrere Peptide, ausgewählt aus der Gruppe, bestehend aus

| (EP004) | PEGRAWAQPGYPWPL, |
|---|---|
| (EP005) | YPWPLYGNEGLGWAG, |
| (EP006) | RGSRPSWGPTDPRRR, |
| (EP007) | GAPLGGAARALAHGV, |
| (EP008) | LAHGVRVLEDGVNYA, |
| (EP010) | AYEVRNVSGVYHVTN, |
| (EP011) | YHVTNDCSNSSIVYE, |
| (EP012) | SIVYEAADMIMHTPG, |
| (EP013) | MHTPGCVPCVRENNS, |
| (EP014) | VQDCNCSIYPGHVSG, |
| (EP015) | PQAVVDMVAGAHWGV, |
| (EP016) | YYSMVGNWAKVLIVM, |
| (EP017) | VDGGGGTTHVTGGAA, |
| (EP019) | GSWHINRTALNCNDS, |
| (EP020) | NCNDSLQTGFLAALF, |
| (EP021) | LAALFYTHRFNSSGC, |
| (EP022) | SCRPIDKFAQGWGPI, |
| (EP023) | GNWFGCTWMNSTGFT, |
| (EP025) | PPCNIGGVGNNTLTC, |
| (EP028) | CGSGPWLTPRCLVDY, |
| (EP029) | TGLIHLHQNIVDVQY, |
| (EP030) | QYFITRAEAHLQVWV, |
| (EP031) | RGGRDAIILLTCAVH, |
| (EP032) | TRVPYFVRAQGLIRA, |
| (EP035) | SDMETKIITWGADTA, |
| (EP036) | ILGLPVSARRGREIL, |
| (EP037) | SLEGQGWRLLAPITA, |
| (EP038) | LTGRDKNQVEGEVQV, |

(EP039)   GEVQVVSTATQSFLA,

(EP040)   ITQMYTNVDQDLVGW,

(EP044)   ADVIPVRRRGDSRGS,

(EP045)   PVSYLKGSSGGPLLC,

(EP046)   HLHAPTGSGKSTKVP,

(EP047)   STKVPAAYAAQGYKV,

(EP049)   NIRTGVRTITTGAPI,

(EP051)   VPHPNIEEVALSNTG,

(EP053)   AIPLEVIKGGRHLIFCHSKR,

(EP054)   GKAIPIEVIKGGRHL,

(EP057)   KLVAMGINAVAYYRGLDVSV,

(EP058)   LSALGLNAVAYYRGL,

(EP059)   YYRGLDVSVIPTSGD,

(EP060)   SVIPTSGDVVVVATDALMTG,

(EP061)   DPTFTIETTTVPQDA,

(EP062)   GIYRFVTPGERPSGM,

(EP063)   RPSGMFDSSVLCECY,

(EP066)   QTKQSGENLPYLVAYQATVC,

(EP068)   ARAQAPPPSWDQMWKCLIRL;

(EP069)   AQAPPPSWDQMWKCL,

(EP070)   DQMWKCLIRLKPTLHGPTPL,

(EP072)   TSTWVLVGGVLAALA,

(EP074)   YIEQGMQLAEQFKQK,

(EP076)   QKLETFWAKHMWNFISGIQY,

(EP077)   MWNFISGIQYLAGLSTLPGN,

(EP078)   NFISGIQYLAGLSTL,

(EP079)   LAGLSTLPGNPAIASLMAFT,

(EP080)   GLSTLPGNPAIASLM,

(EP081)   QTLLFNILGGWVAAQLAAPG,

(EP083)   AAQLAPPSAASAFVG,

(EP084)   SAFVGAGIAGAAVGS,

(EP086)   KVMSGEMPSTEDLVN,

(EP090) LKRLHQWINEDCSTP,

(EP092) LRDVWDWICTVLTDF,

(EP093) VLTDFKTWLQSKLLP,

(EP094) SKLLPRLPGVPFFSC,

(EP095) GVWRGDGIMQTTCPC,

(EP097) TTCPCGAQITGHVKN,

(EP099) NYSRALWRVAAEEYV,

(EP101) AEEYVEVTRVGDFHY,

(EP104) PAPEFFTEVDGVRLH,

(EP106) GVRLHRYAPACKPLL,

(EP108) FQVGLNQYLVGSQLP,

(EP110) DPSHITAETAKRRLA,

(EP111) KRRLARGSGVPPSLA,

(EP112) PPSLASSSASQLSAP,

(EP114) QLSAPSLKATCTTHH,

(EP116) VILDSFDPLRAEEDE,

(EP122) QASDDGDKGSDVESY,

(EP125) ETPIDTTIMAKNEVF,

(EP126) KNEVFCVQPEKGGRK,

(EP127) KGGRKPARLIVFPDL,

(EP128) TLPQAVMGSSYGFQY,

(EP129) TRCFDSTVTENDIRV,

(EP130) QCCDLAPEARQAIRS,

(EP131) YIGGPLTNSKGQNCG,

(EP133) VYYLTRDPTTPLARA,

(EP134) QIIQRLHGLSAFSLH,

(EP135) AFSLHSYSPGEINRV,

(EP137) KLGVPPLRVWRHRAR,

(EP138) CGKYLFNWAVRTKLK, und

(EP139) RTKLKLTPIPAASQL sowie Derivate davon mit gleicher oder ähnlicher Spezifität.

7. CD4<sup>+</sup> T-Lymphozyten spezifische HCV-Epitope nach Anspruch 4, umfassend ein oder mehrere Peptide, ausgewählt aus der Gruppe, bestehend aus

| | |
|---|---|
| (EP001) | DVKFPGGGQIVGGVY, |
| (EP003) | RRQPIPKARRPEGRA, |
| (EP009) | GVNYATGNLPGCSFS, |
| (EP018) | GFTSLFSPGASQKIQ, |
| (EP024) | STGFTKTCGGPPCNI, |
| (EP026) | NTLTCPTDCFRKHPE, |
| (EP027) | RKHPEATYTKCGSGP, |
| (EP033) | GLIRACMLVRKVAGG, |
| (EP034) | AFMKLAALTGTYVYD, |
| (EP041) | DLVGWQAPPGARSLT, |
| (EP050) | TGAPITYSTYGKFLA, |
| (EP052) | LSNTGEIPFYGKAIP, |
| (EP056) | KKKCDELAAKLSALG, |
| (EP064) | TTVRLRAYMNTPGLPVCQ, |
| (EP065) | VRLRAYLNTPGLPVC, |
| (EP067) | YLVAYQATVCARAQAPPPSW, |
| (EP073) | ILSGRPAVIPDREVL, |
| (EP075) | APVVESKWRALETFW, |
| (EP087) | EDLVNLLPAILSPGA, |
| (EP096) | DGIMQTTCPCGAQITGHVKN, |
| (EP100) | LWRVAAEEYVEVTRVGDFHY, |
| (EP107) | REEVTFQVGLNQYLVGSQLP, |
| (EP115) | SLKATCTTHHSYNLDSPDAD, |
| (EP117) | FDPLRAEEDEREVSVAAEIL, |
| (EP119) | PIWARPDYNPPLLESWKDPD, |
| (EP121) | YVPPVVHGCPLPPTKAPPIP, |
| (EP124) | WTGALITPCAAEESKLPINA, |
| (EP132) | GQNCGYRRCRASGVL, |

(EP136)     EINRVASCLRKLGVP, und

(EP140)     VGVGIYLLPNR sowie Derivate hiervon mit gleicher oder ähnlicher Spezifität.

8.    CD4$^+$ T-Lymphozyten spezifische HCV-Epitope nach Anspruch 5, umfassend ein oder mehrere Peptide, ausgewählt aus der Gruppe, bestehend aus

(EP002)     GPRLGVRATRKTSER,

(EP042)     ARSLTPCTCGSSDLY,

(EP043)     SSDLYLVTRHADVIP,

(EP048)     QGYKVLVLNPSVAAT,

(EP055)     GGRHLIFCHSKKKCD,

(EP071)     MWKCLIRLKPTLHGP,

(EP082)     LLFNILGGWVAAQLA,

(EP085)     VLVDILAGYGAGVAG,

(EP088)     THYVPESDAAARVTQILSSL,

(EP089)     TITQLLKRLHQWINEDCSTP,

(EP091)     CSGSWLRDVWDWICTVLTDF,

(EP098)     GAQITGHVKNGSMRIVGPKT,

(EP102)     EVTRVGDFHYVTGMTTDNVK,

(EP103)     CPCQVPAPEFFTEVDGVRLH,

(EP105)     FTEVDGVRLHRYAPACKPLL,

(EP109)     TSMLTDPSHITAETAKRRLA,

(EP113)     SSSASQLSAPSLKATCTTHH,

(EP118)     REVSVAAEILRKSRKFPPAM,

(EP120)     PLLESWKDPDYVPPVVHGCP, und

(EP123)     DVVCCSMSYTWTGALITPCA sowie Derivate hiervon mit gleicher oder ähnlicher Spezifität.

9.    CD4$^+$ T-Lymphozyten spezifische HCV-Epitope mit einem oder mehreren in den Ansprüchen 6 bis 8 genannten Sequenzen.

10.   Impfstoff, mindestens ein HCV-Epitop nach einem der vorstehenden Ansprüche umfassend.

11.   Impfstoff nach Anspruch 10, zusätzlich mindestens einen Hilfsstoff, ausgewählt aus der Gruppe, bestehend aus fowlpoxvirus, modifiziertem Vakziniavirus Ankara, Virosomen, Transvax und anderen die Immunreaktion verstärkenden Stoffen, umfassend.

**12.** Impfstoff nach einem der Ansprüche 10 oder 11 in Form einer Injektionslösung.

**13.** HCV-Epitop nach einem der Ansprüche 1 bis 8 als Arzneimittel.

**14.** Verwendung mindestens eines HCV-Epitops nach einem der Ansprüche 1 bis 8 zur Herstellung einer Zusammensetzung zur Prophylaxe und/oder Therapie einer Hepatitis C Infektion.

**15.** Verwendung mindestens eines HCV-Epitop nach einem der Ansprüche 1 bis 8 in der Diagnose einer Hepatitis C Infektion.

**16.** Diagnostischer Kit, mindestens eines der Epitope nach einem der Ansprüche 1 bis 8 umfassend.

**17.** cDNA, eines der HCV-Epitope nach einem der Ansprüche 1 bis 8 kodierend.

**18.** Impfstoff, mindestens eine cDNA nach einem der Ansprüche 1 bis 8 umfassend.

EP 1 357 127 A1

## EUROPÄISCHER TEILRECHERCHENBERICHT

Europäisches Patentamt

der nach Regel 45 des Europäischen Patentübereinkommens für das weitere Verfahren als europäischer Recherchenbericht gilt

**Nummer der Anmeldung**

EP 02 00 8033

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X | US 6 183 949 B1 (IHLENFELDT HANS GEORG ET AL) 6. Februar 2001 (2001-02-06) Sequenz #32 * das ganze Dokument * --- | 6,9-18 | C07K14/18 A61K39/29 G01N33/576 G01N33/68 C12N15/51 A61K48/00 A61P31/14 |
| X | DATABASE TREMBL [Online] Polyprotein (Fragment) from Hepatitis C virus, 1. November 1996 (1996-11-01) retrieved from EBI Database accession no. q68529 XP002208337 | 6,9,17 | |
| Y | * Zusammenfassung * --- | 10-18 | |
| Y | WO 02 26785 A (DIEPOLDER HELMUT ;GERLACH JOERN TILMAN (DE); IMMUSYSTEMS GMBH (DE)) 4. April 2002 (2002-04-04) * das ganze Dokument * ----- | 10-18 | |

| RECHERCHIERTE SACHGEBIETE (Int.Cl.7) |
|---|
| C07K C12N A61K |

### UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| MÜNCHEN | 5. August 2002 | Rutz, B |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie, übereinstimmendes Dokument

EPO FORM 1503 03.82 (P04C09)

52

**Europäisches Patentamt**

**UNVOLLSTÄNDIGE RECHERCHE ERGÄNZUNGSBLATT C**

Nummer der Anmeldung

EP 02 00 8033

Unvollständig recherchierte Ansprüche:
      10-18

Nicht recherchierte Ansprüche:
      1-5

Grund für die Beschränkung der Recherche:

Die geltenden Patentansprüche 1-5 und 10-18 sind auf ein Produkt, das mittels folgender Parameter definiert wird, zu beziehen:
 P1: CD4+ T-Lymphocyten spezifische HCV-Epitope
 P2: mit einem Impact factor (IF) > Mittelwert (MW) wobei IF [...] entspricht [...] und MW der Mittelwert aller Impact factoren ist.
Der Impact factor wird über eine in Anspruch 1 angeführte Formel 1 definiert. Diese liefert einen relativen statistischen Wert, der den Stimulationsindex (SI) des beanspruchten Epitops in Verhältnis zu den Stimulationsindices der übrigen getesteten Epitope setzt. Dabei ist die Auswahl der getesteten Epitope mit Ausnahme ihrer Anzahl (m>=15) nicht näher definiert.
Da es sich bei dem Parameter P2 lediglich um einen relativen statistische Wert handelt, muss die Verwendung dieses Parameters im gegebenen Zusammenhang als Mangel an Klarheit im Sinne von Art. 84 EPÜ erscheinen. Es ist unmöglich, die vom Anmelder gewählten Parameter mit dem zu vergleichen, was der Stand der Technik hierzu offenbart. Der Mangel an Klarheit ist dergestalt, daß er eine sinnvolle vollständige Recherche unmöglich macht. Daher wurde die Recherche beschränkt auf die Teile mit Bezug auf Sequenzen wie sie im eingereichten Sequenzprotokoll niedergelegt wurden.

**Europäisches**

**Patentamt**

Nummer der Anmeldung

EP 02 00 8033

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

Ansprüche 6 und 9-18 (alle teilweise)

**Europäisches Patentamt**

**MANGELNDE EINHEITLICHKEIT DER ERFINDUNG ERGÄNZUNGSBLATT B**

Nummer der Anmeldung

EP 02 00 8033

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Erfindung 1: Ansprüche 6 und 9-18 (alle teilweise)

CD4+ T-Lymphozyten spezifisches HCV-Epitop mit Sequenz nach SEQ ID NO: 4, Impfstoffe, Arzneimittel, cDNA, Verwendung in Diagnose

Erfindungen 2-140: Ansprüche 6, 7, 8 und 9-18 (alle teilweise)

CD4+ T-Lymphozyten spezifisches HCV-Epitop mit Sequenz nach SEQ ID NO: 1-3 und 5-140, Impfstoffe, Arzneimittel, cDNA, Verwendung in Diagnose

## ANHANG ZUM EUROPÄISCHEN RECHERCHENBERICHT
## ÜBER DIE EUROPÄISCHE PATENTANMELDUNG NR.

EP 02 00 8033

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-08-2002

| Im Recherchenbericht angeführtes Patentdokument | Datum der Veröffentlichung | Mitglied(er) der Patentfamilie | Datum der Veröffentlichung |
|---|---|---|---|
| US 6183949 B1 | 06-02-2001 | DE 4209215 A1 | 07-01-1993 |
| | | AT 171710 T | 15-10-1998 |
| | | AU 652851 B2 | 08-09-1994 |
| | | AU 2197392 A | 11-02-1993 |
| | | CA 2089576 A1 | 05-01-1993 |
| | | DE 59209512 D1 | 05-11-1998 |
| | | WO 9301210 A2 | 21-01-1993 |
| | | EP 0551460 A1 | 21-07-1993 |
| | | EP 0881231 A2 | 02-12-1998 |
| | | ES 2123558 T3 | 16-01-1999 |
| | | JP 2774872 B2 | 09-07-1998 |
| | | JP 5506462 T | 22-09-1993 |
| | | KR 9710925 B1 | 02-07-1997 |
| | | US 6592871 B1 | 15-07-2003 |
| | | ZA 9204950 A | 03-01-1994 |
| WO 0226785 A | 04-04-2002 | EP 1195381 A1 | 10-04-2002 |
| | | AU 2356902 A | 08-04-2002 |
| | | WO 0226785 A2 | 04-04-2002 |

Für nähere Einzelheiten zu diesem Anhang : siehe Amtsblatt des Europäischen Patentamts, Nr.12/82

EPO FORM P0461